# EUROPEAN PATENT APPLICATION

(11) **EP 4 349 846 A1**
(43) Date of publication of application: **10.04.2024**
(21) Application number: 22816214.5
(22) Date of filing: 02.06.2022
(51) Int. Cl.: C07H 23/00

(54) **CHIMERIC NUCLEIC ACID OLIGOMER INCLUDING PHOSPHOROTHIOATE AND BORANOPHOSPHATE, AND METHOD FOR PRODUCING SAME**

(30) Priority: 02.06.2021 JP 2021092873
(71) Applicant: TOKYO UNIVERSITY OF SCIENCE FOUNDATION, Tokyo 162-8601 (JP)
(72) Inventor: WADA Takeshi, Tokyo 162-8601 (JP); SATO Kazuki, Tokyo 162-8601 (JP); TAKAHASHI Yuhei, Tokyo 162-8601 (JP)
(74) Representative: Isarpatent
(86) International application number: PCT/JP2022/022556
(87) International publication number: WO 2022/255469

(57) **Abstract**

Provided are a novel nucleic acid oligomer and a method for producing the same. The nucleic acid oligomer according to the present invention includes a nucleotide unit represented by general formula (1) and a nucleotide unit represented by general formula (2), and may or may not include a nucleotide unit represented by general formula (3). In general formulas (1), (2), and (3): R1 represents a hydrogen atom, a hydroxyl group, an alkoxy group, an alkenyloxy group, an acyloxy group, a trialkylsilyloxy group, an alkoxyalkoxy group, a haloalkoxyalkoxy group, or a halogenyl group, and R² represents a hydrogen atom, or R¹ and R² bond together to form a five- or more-membered ring optionally having a substituent and optionally having a hetero atom; Bs represents a nucleobase selected from the group consisting of adenine, guanine, cytosine, 5-methylcytosine, thymine, and uracil, the nucleobase optionally having a nucleobase protecting group; and X⁺ represents a counter cation.

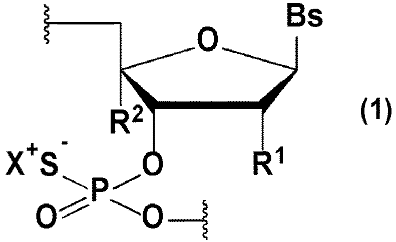

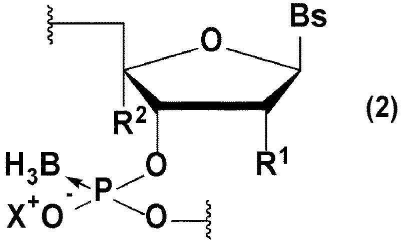

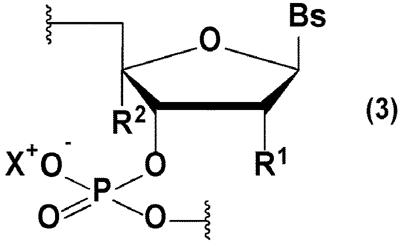

## Description

### TECHNICAL FIELD

The present invention relates to a nucleic acid oligomer and a method for producing the nucleic acid oligomer.

### BACKGROUND ART

An antisense molecule having a base sequence complementary to a target nucleic acid can form a duplex complementarily to the target nucleic acid and inhibit protein production from the target nucleic acid. When a disease-related gene is selected as the target nucleic acid, the antisense molecule directly acts on the disease-related gene, so the antisense molecule has received a lot of attention as an effective medicament for gene therapy.

The antisense molecule (nucleic acid oligomer) is required to have a cell membrane permeability, a nuclease resistance, a chemical stability in vivo (for example, in an environment of pH 7.4), and a property of forming a stable duplex only with a specific base sequence primarily from the viewpoint of efficient inhibition of production of a target protein. For example, a nucleic acid oligomer obtained using a phosphorothioate compound (hereinafter referred to as "phosphorothioate type nucleic acid oligomer) (Non-Patent Document 1) and a nucleic acid oligomer obtained using a boranophosphate compound (hereinafter referred to as "boranophosphate type nucleic acid oligomer) (Non-Patent Document 2) have been known as the antisense molecule.

Non-Patent Document 1: Nucleic Acids Research, 2010, Vol.38, No.20, pp.7100-7111
Non-Patent Document 2: Chem. Rev., 2007, Vol.107, pp.4746-4796

### DISCLOSURE OF THE INVENTION

### Problems to be Solved by the Invention

An object of the present invention is to provide a novel nucleic acid oligomer and a method for producing the nucleic acid oligomer.

### Means for Solving the Problems

Specific means for solving the above-mentioned object include the following embodiments.
<1> A nucleic acid oligomer comprising:
   a nucleotide unit represented by General Formula (1) below;
   a nucleotide unit represented by General Formula (2) below; and
   optionally a nucleotide unit represented by General Formula (3) below: 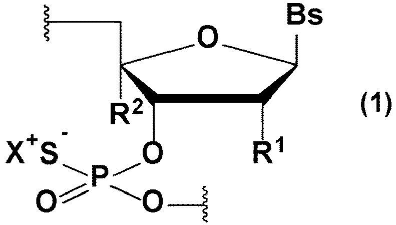 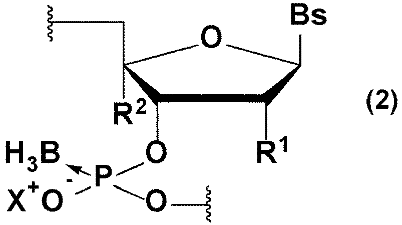 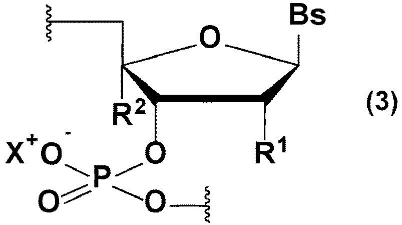
   wherein R¹ denotes a hydrogen atom, a hydroxy group, an alkoxy group, an alkenyloxy group, an acyloxy group, a trialkylsilyloxy group, an alkoxyalkoxy group, a haloalkoxyalkoxy group, or a halogenyl group, R² denotes a hydrogen atom, or R¹ and R² bind to each other to form a five or more membered ring optionally having a substituent and optionally having a hetero atom, Bs denotes a nucleic acid base selected from the group consisting of adenine, guanine, cytosine, 5-methylcytosine, thymine, and uracil, the nucleic acid base optionally has a protecting group of the nucleic acid base, and X⁺ denotes a counter cation.
<2> The nucleic acid oligomer according to aspect <1>, wherein R¹ denotes a hydrogen atom, a hydroxy group, an alkoxy group, an alkenyloxy group, an acyloxy group, a trialkylsilyloxy group, an alkoxyalkoxy group, a haloalkoxyalkoxy group, or a halogenyl group and R² denotes a hydrogen atom, or R¹ and R² bind to each other to form a divalent group represented by General Formula (4) below: 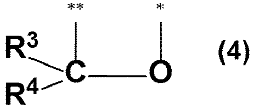 wherein R³ and R⁴ each independently denote a hydrogen atom or an alkyl group having 1 to 10 carbon atoms or bind to each other to form a ring, * and ** denote an atomic bond, an atomic bond represented by * is bonded to a carbon atom to which R¹ directly binds in General Formula (1), (2), or (3), and an atomic bond represented by ** is bonded to a carbon atom to which R² directly binds in General Formula (1), (2), or (3) .
<3> A method for producing the nucleic acid oligomer according to aspect <1> or <2>, the method comprising: sequentially condensing a nucleotide monomer selected from the group consisting of a compound represented by General Formula (5) below and a compound represented by General Formula (6) below or a nucleotide monomer selected from the group consisting of a compound represented by General Formula (5) below, a compound represented by General Formula (6) below, and a compound represented by General Formula (7) below to obtain a precursor nucleic acid oligomer comprising a nucleotide unit represented by General Formula (8) below and a nucleotide unit represented by General Formula (9) below and optionally comprising a nucleotide unit represented by General Formula (10) below; and
   oxidizing the precursor nucleic acid oligomer with an oxidizing agent to obtain the nucleic acid oligomer according to aspect <1>: 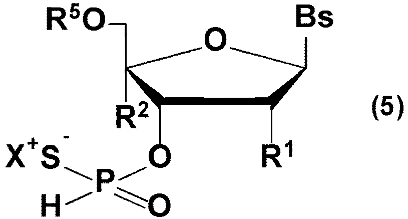 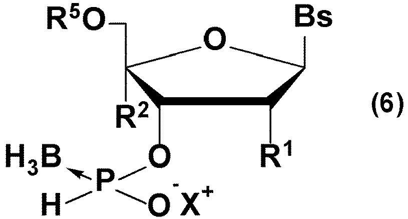 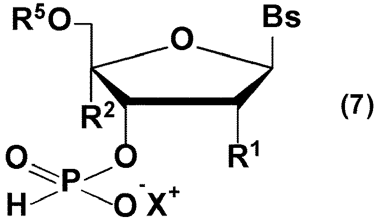
   wherein R¹, R², Bs, and X⁺ are as described above and R⁵ denotes a hydrogen atom or a protecting group of a hydroxy group, 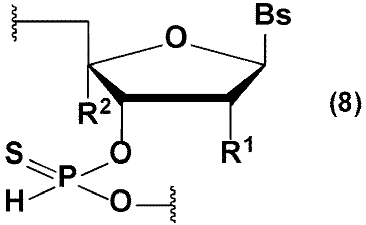 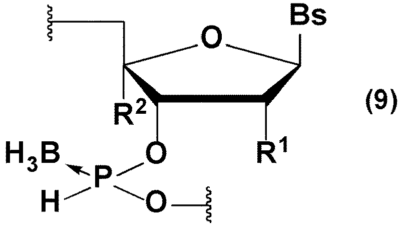 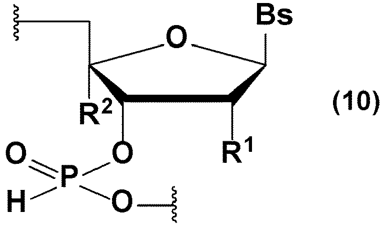
   wherein R¹, R², and Bs are as described above.
<4> The method according to aspect <3>, wherein at least the condensing is performed by a reaction using a solid-phase support.

### Effects of the Invention

According to the present invention, a novel nucleic acid oligomer and a method for producing the nucleic acid oligomer can be provided.

### BRIEF DESCRIPTION OF THE DRAWINGS

FIGs. 1A to 1F are diagrams showing charts of reverse-phase HPLC for Comparative Synthesis Examples or Synthesis Examples. FIG. 1A: Comparative Synthesis Example 1, FIG. 1B: Comparative Synthesis Example 2, FIG. 1C: Synthesis Example 2, FIG. 1D: Synthesis Example 3, FIG. 1E: Synthesis Example 6, and FIG. 1F: Synthesis Example 7.
FIGs. 2A and 2B are diagrams showing charts of reverse-phase HPLC for the nucleic acid oligomer of Example 1. FIG. 2A: before separation and purification and FIG. 2B: after separation and purification.
FIGs. 3A and 3B are diagrams showing charts of reverse-phase HPLC for the nucleic acid oligomer of Example 2. FIG. 3A: before separation and purification and FIG. 3B: after separation and purification.
FIGs. 4A and 4B are diagrams showing charts of reverse-phase HPLC for the nucleic acid oligomer of Example 3. FIG. 4A: before separation and purification and FIG. 4B: after separation and purification.
FIGs. 5A and 5B are diagrams showing charts of reverse-phase HPLC for the nucleic acid oligomer of Example 4. FIG. 5A: before separation and purification and FIG. 5B: after separation and purification.
FIG. 6 is a diagram showing a chart of reverse-phase HPLC for Synthesis Example 12.
FIG. 7 is a diagram showing a chart of reverse-phase UHPLC for the nucleic acid oligomer of Example 5.
FIGs. 8A and 8B are diagrams showing charts of reverse-phase HPLC for the nucleic acid oligomer of Example 6. FIG. 8A: before separation and purification and FIG. 8B: after separation and purification.

### PREFERRED MODE FOR CARRYING OUT THE INVENTION

Embodiments of the present invention will now be described. It is noted that the present invention is not limited to the following embodiments. The term "step" as used herein includes not only an independent step but also a step clearly indistinguishable from another step as long as a desired purpose of the step can be achieved. As used herein, the term "to" described between numerical values shall indicate a range that includes the numerical values described before and after it as the minimum and maximum values, respectively. Moreover, unless otherwise specified, an amount of a component in a composition herein means, when a plurality of substances corresponding to the component is present in the composition, a total amount of the plurality of substances present in the composition.

### <Nucleic acid oligomer>

A nucleic acid oligomer according to the present embodiment includes a nucleotide unit represented by General Formula (1) below and a nucleotide unit represented by General Formula (2) below and optionally includes a nucleotide unit represented by General Formula (3) below.

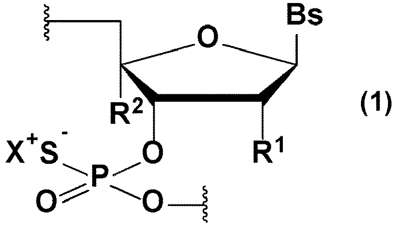

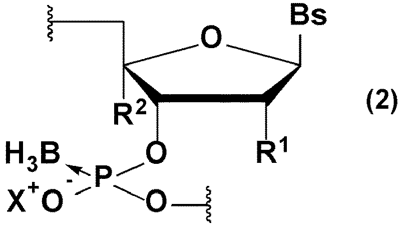

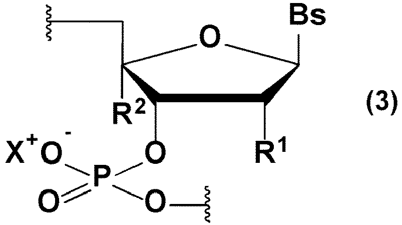

In General Formulae (1), (2), and (3), R¹ denotes a hydrogen atom, a hydroxy group, an alkoxy group, an alkenyloxy group, an acyloxy group, a trialkylsilyloxy group, an alkoxyalkoxy group, a haloalkoxyalkoxy group, or a halogenyl group, R² denotes a hydrogen atom, or R¹ and R² bind to each other to form a five or more membered ring optionally having a substituent and optionally having a hetero atom, Bs denotes a nucleic acid base selected from the group consisting of adenine, guanine, cytosine, 5-methylcytosine, thymine, and uracil, the nucleic acid base optionally has a protecting group of the nucleic acid base, and X⁺ denotes a counter cation.

A nucleic acid oligomer including the nucleotide unit represented by General Formula (1) tends to have an excellent affinity to a target RNA, an excellent RNase H-inducing activity, and an excellent nuclease resistance, and especially excellent blood retentivity, but is likely to be highly toxic. A nucleic acid oligomer including the nucleotide unit represented by General Formula (2) tends to have an excellent affinity to a target RNA, an excellent RNase H-inducing activity, and an especially excellent nuclease resistance, and is less likely to be highly toxic, but has not been confirmed to tend to have excellent blood retentivity. A nucleic acid oligomer including the nucleotide unit represented by General Formula (3) tends to have an especially excellent affinity to a target RNA and an especially excellent RNase H-inducing activity, and is less likely to be highly toxic, but is likely to be less nuclease resistant and have low blood retentivity. The nucleic acid oligomer according to the present embodiment includes the nucleotide unit represented by General Formula (1) and the nucleotide unit represented by General Formula (2) or includes the nucleotide unit represented by General Formula (1), the nucleotide unit represented by General Formula (2), and the nucleotide unit represented by General Formula (3) and thus is expected to be less toxic while maintaining an affinity to a target RNA, an RNase H-inducing activity, a nuclease resistance, and blood retentivity at a high level.

A length (base length) of the nucleic acid oligomer according to the present embodiment is not particularly limited as long as the nucleic acid oligomer has two or more bases and a desired length can be appropriately selected depending on a type, length, and the like of a target nucleic acid. The length of the nucleic acid oligomer is not particularly limited, but is preferably 8 to 50 bases, more preferably 10 to 30 bases, and further preferably 10 to 21 bases from the viewpoint of application to an antisense drug.

A base sequence of the nucleic acid oligomer is set based on a base sequence of a target nucleic acid. From the viewpoint of a duplex forming ability, the base sequence of the nucleic acid oligomer is appropriately selected so as to be a base sequence complementary to that of the target nucleic acid, but, in some cases, may be a base sequence including one or more different bases from that of the target nucleic acid. Furthermore, proportions of the nucleotide unit represented by General Formula (1), the nucleotide unit represented by General Formula (2), and the nucleotide unit represented by General Formula (3) in the nucleic acid oligomer are appropriately selected so as to be less toxic while maintaining an affinity to a target RNA, an RNase H-inducing activity, a nuclease resistance, and blood retentivity at a high level.

The alkoxy group represented by R¹ is preferably an alkoxy group having 1 to 12 carbon atoms and more preferably an alkoxy group having 1 to 6 carbon atoms. Examples thereof include a methoxy group, an ethoxy group, an n-propoxy group, an i-propoxy group, an n-butoxy group, a sec-butoxy group, a tert-butoxy group, and an n-pentyloxy group.

Examples of the alkenyloxy group represented by R¹ include a vinyloxy group, an allyloxy group, a 1-propenyloxy group, an isopropenyloxy group, a 2-methyl-1-propenyloxy group, a 2-methylallyloxy group, and a 2-butenyloxy group.

Examples of the acyloxy group represented by R¹ include an alkyl-carbonyloxy group having 1 to 6 carbon atoms (e.g., a methylcarbonyloxy group, an ethylcarbonyloxy group, etc.) and an aryl-carbonyloxy having 6 to 10 carbon atoms (e.g., a benzoyloxy group).

Examples of the trialkylsilyloxy group represented by R¹ include a trimethylsilyloxy group and a triethylsilyloxy group.

Examples of the alkoxyalkoxy group represented by R¹ include a methoxymethoxy group, a methoxyethoxy group, an ethoxymethoxy group, and an ethoxyethoxy group.

Examples of the haloalkoxyalkoxy group represented by R¹ include a (2-fluoro)ethoxymethoxy group, a (2,2-difluoro)ethoxymethoxy group, a (2-chloro)methoxyethoxy group, and a (2,2-dichloro)ethoxymethoxy group.

Examples of the halogenyl represented by R¹ include a fluoro group, a chloro group, and a bromo group, with the fluoro group being preferred.

From the viewpoint of a duplex forming ability, R¹ is preferably a hydrogen atom, a hydroxy group, an alkoxy group, a trialkylsilyloxy group, an alkoxyalkoxy group, a haloalkoxyalkoxy group, and a halogenyl group, among others.

R¹ and R² may bind to each other to form a five or more-membered ring optionally having a substituent and optionally having a hetero atom. Examples of the substituent include a substituent bound to an atom constituting the ring, specifically, an alkyl group optionally having a substituent, an oxo group (=O), etc. Examples of the alkyl group include a methyl group and an ethyl group. When the alkyl group has a substituent, examples of the substituent include a halogenyl group such as a fluoro group, a chloro group, and a bromo group, an amino group, and an imino group. Examples of the hetero atom include an oxygen atom, a nitrogen atom, and a sulfur atom. A number of members in the ring is preferably 5 to 10, more preferably 5 to 8, and further more preferably 5 to 7.

From the viewpoint of a duplex forming ability, R¹ and R² preferably bind to each other to form a divalent group represented by General Formula (4) below.

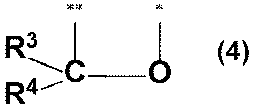

In General Formula (4), R³ and R⁴ each independently denote a hydrogen atom or an alkyl group having 1 to 10 carbon atoms or bind to each other to form a ring, * and ** denote an atomic bond, an atomic bond represented by * is bonded to a carbon atom to which R¹ directly binds in General Formula (1), (2), or (3), and an atomic bond represented by ** is bonded to a carbon atom to which R² directly binds in General Formula (1), (2), or (3).

Examples of the alkyl group represented by R³ or R⁴ include a linear or branched alkyl group having 1 to 10 carbon atoms, specifically, for example, a methyl group, an ethyl group, an n-propyl group, an i-propyl group, an n-butyl group, a t-butyl group, an n-hexyl group, and an n-octyl group, etc. When R³ and R⁴ bind to each other to form a ring, specific examples of the ring include a cyclopropane ring, a cyclobutane ring, a cyclohexane ring, etc.

When R¹ and R² bind to each other to form a five or more-membered ring optionally having a substituent and optionally having a hetero atom, specific examples of the ring include rings shown below: 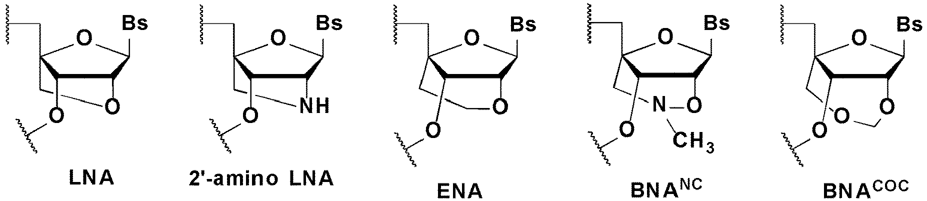 in which Bs is as described above.

Bs denotes a nucleic acid base selected from the group consisting of adenine, guanine, cytosine, 5-methylcytosine, thymine, and uracil, the nucleic acid base optionally has a protecting group of the nucleic acid base. When the nucleic acid base has a protecting group, a type of the protecting group is not particularly limited. Examples of the protecting group include a benzyl group, a benzoyl group, a 4-methoxybenzoyl group, an acetyl group, a propionyl group, a butyryl group, an isobutyryl group, a phenylacetyl group, a phenoxyacetyl group, a chloroacetyl group, a 4-tert-butylphenoxyacetyl group, a 4-isopropylphenoxyacetyl group, a (dimethylamino)methylene group, etc.

X⁺ denotes a counter cation. Examples of the counter cation include an ammonium ion, a cation derived from an organic amine compound, or a metal cation, etc. Examples of the cation derived from an organic amine compound include a cation derived from a tertiary alkylamine compound or a cation derived from a quaternary ammonium compound from the viewpoints of solubility in an organic solvent and volatility of the counter cation. Examples of the cation derived from a tertiary alkylamine compound include a cation derived from triethylamine HNEt₃⁺ in which Et denotes an ethyl group and a cation derived from 1,8-diazabicyclo[5.4.0]undecene. Examples of the cation derived from a quaternary ammonium compound include a tetrabutylammonium ion. Examples of the metal cation include Na⁺, Li⁺, K⁺, etc.

The nucleic acid oligomer according to the present embodiment may be a nucleic acid oligomer having a nucleotide unit other than the nucleotide unit represented by General Formula (1), the nucleotide unit represented by General Formula (2), and the nucleotide unit represented by General Formula (3). A total proportion of the nucleotide unit represented by General Formula (1), the nucleotide unit represented by General Formula (2), and the nucleotide unit represented by General Formula (3) in the nucleic acid oligomer is not particularly limited, but from the viewpoint of a duplex forming ability, preferably 40 to 100% by mole, more preferably 50 to 100% by mole, further more preferably 60 to 100% by mole, and particularly preferably 70 to 100% by mole relative to all nucleotide units in the nucleic acid oligomer.

The nucleic acid oligomer according to the present embodiment has a hydroxy group or a protecting group of a hydroxy group at a 5' end and a hydroxy group or a protecting group of a hydroxy group at a 3' end. Examples of the protecting group of a hydroxy group include an acetyl group, a phenylacetyl group, a phenoxyacetyl group, a chloroacetyl group, a pivaloyl group, a benzyl group, a 4-methoxybenzyl group, a benzoyl group, a 4-methoxybenzoyl group, a triphenylmethyl group, a 4,4'-dimethoxytrityl (DMTr) group, a 4-methoxytrityl (MMTr) group, a 9-phenylxanthenyl group, a t-butoxycarbonyl group, a trimethylsilyl group, a t-butyldimethylsilyl group, a t-butyldiphenylsilyl group, a cyanomethoxymethyl group, a 2-(cyanoethoxy)ethyl group, a cyanoethoxymethyl group, etc.

### <Method for producing nucleic acid oligomer>

A method for producing a nucleic acid oligomer according to the present embodiment includes
sequentially condensing a nucleotide monomer selected from the group consisting of a compound represented by General Formula (5) below and a compound represented by General Formula (6) below or a nucleotide monomer selected from the group consisting of a compound represented by General Formula (5) below, a compound represented by General Formula (6) below, and a compound represented by General Formula (7) below to obtain a precursor nucleic acid oligomer including a nucleotide unit represented by General Formula (8) below and a nucleotide unit represented by General Formula (9) below and optionally including a nucleotide unit represented by General Formula (10) below; and
oxidizing the precursor nucleic acid oligomer with an oxidizing agent to obtain the nucleic acid oligomer according to the present embodiment.

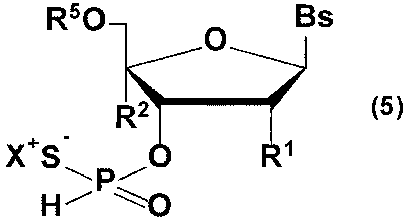

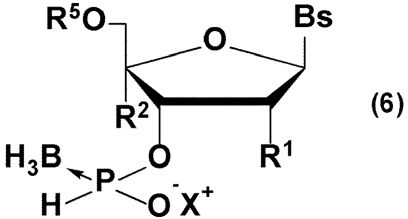

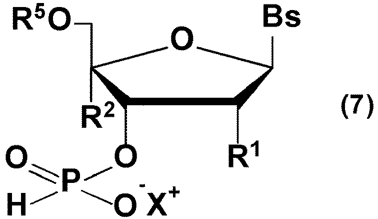

In General Formula (5), (6), and (7), R¹, R², Bs, and X⁺ are as described above and R⁵ denotes a hydrogen atom or a protecting group of a hydroxy group. Examples of the protecting group of a hydroxy group include an acetyl group, a phenylacetyl group, a phenoxyacetyl group, a chloroacetyl group, a pivaloyl group, a benzyl group, a 4-methoxybenzyl group, a benzoyl group, a 4-methoxybenzoyl group, a triphenylmethyl group, a 4,4'-dimethoxytrityl (DMTr) group, a 4-methoxytrityl (MMTr) group, a 9-phenylxanthenyl group, a t-butoxycarbonyl group, a trimethylsilyl group, a t-butyldimethylsilyl group, a t-butyldiphenylsilyl group, a cyanomethoxymethyl group, a 2-(cyanoethoxy)ethyl group, a cyanoethoxymethyl group, etc. Among others, a 4,4'-dimethoxytrityl group is preferred since it can be readily removed under an acidic condition and is suitable for chain elongation in solid-phase synthesis.

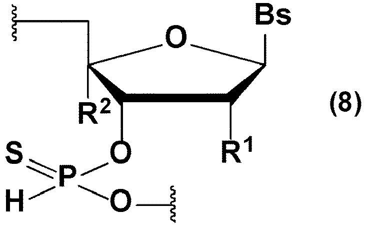

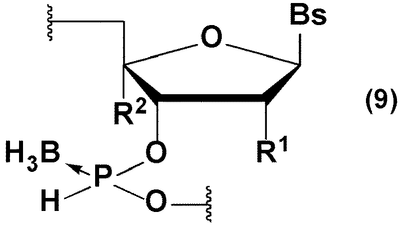

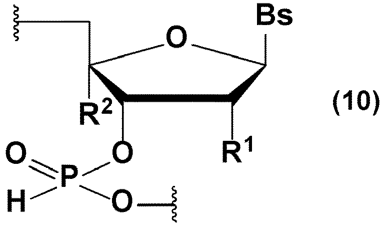

In General Formula (8), (9), and (10), R¹, R², and Bs are as described above.

In the condensing step, a nucleotide monomer selected from the group consisting of a compound represented by General Formula (5) and a compound represented by General Formula (6) or a nucleotide monomer selected from the group consisting of a compound represented by General Formula (5), a compound represented by General Formula (6), and a compound represented by General Formula (7) are sequentially condensed to obtain a precursor nucleic acid oligomer including a nucleotide unit represented by General Formula (8) and a nucleotide unit represented by General Formula (9) and optionally including a nucleotide unit represented by General Formula (10). During the condensing step, a hydroxy group bound to a carbon atom at position 5' in a ribose structure in a nucleoside structure of the nucleic acid oligomer is condensed with a phosphate moiety of the monomer.

In the condensing step, a condensing agent may be used. Examples of the condensing agent include N,N-bis(2-oxo-3-oxazolidinyl)phosphinate chloride, 3-nitro-1,2,4-triazol-1-yl-tris(pyrrolidin-1-yl)phosphonium hexafluorophosphate (PyNTP), or 1,3,-dimethyl-2-(3-nitro-1,2,4-triazol-1-yl)-2-pyrrolidin-1-yl-1,3,2-diazaphosphoridium hexafluorophosphate (MNTP), etc. These condensing agents are suitable for synthesizing a long nucleic acid oligomer since a side reaction with a hydroxy group on a solid-phase support is less likely to occur. The compound represented by General Formula (5) has an oxygen atom and a sulfur atom as nucleophilic atoms in a molecule. When the oxygen atom reacts with the condensing agent, a desired reaction proceeds, but when the sulfur atom reacts with the condensing agent, a side reaction occurs. From the viewpoint of prevention of the side reaction, PyNTP is preferably used as the condensing agent when the compound represented by General Formula (5) is condensed. When the compound represented by General Formula (6) or the compound represented by General Formula (7) is condensed, MNTP is preferably used as the condensing agent from the viewpoint of rapid completion of the reaction.

The condensing step may also be performed in the presence of a basic compound. Examples of the basic compound include 1,8-bis(dimethylamino)naphthalene (DMAN), 2,2,6,6-tetramethylpiperidine (TMP), diisopropylethylamine (DIPEA), 2,6-lutidine, 2,4,6-trimethylpyridine, pyridine, quinoline, etc. Among them, from the viewpoint of prevention of the side reaction, quinoline is preferably used when a compound represented by General Formula (5) in which Bs is adenine, cytosine, or 5-methylcytosine is condensed, and 2,6-lutidine is preferably used when the compound represented by General Formula (6) or the compound represented by General Formula (7) is condensed. Note that, from the viewpoint of prevention of the side reaction, the condensing step is preferably performed in the absence of the basic compound when a compound represented by General Formula (5) in which Bs is guanine, thymine, or uracil is condensed.

The condensing reaction in the condensing step can be performed, for example, on ice, i.e., 0°C or more and room temperature, i.e., 25°C or less, preferably 20°C or more and 25°C or less for 1 min or more and several hours or less, preferably 3 min or more and 1 hour or less.

A reaction solvent may be, for example, an inert solvent, etc. The inert solvent may be, for example, acetonitrile, etc.

In the method for producing a nucleic acid oligomer according to the present embodiment, at least the condensing step is preferably performed by a reaction using a solid-phase support (solid-phase method). Specifically, both the condensing step and the oxidizing step may be performed by a reaction using a solid-phase support, or the condensing step may be performed by a reaction using a solid-phase support and the oxidizing step may be performed by another method, for example, a reaction in a liquid phase. When the nucleic acid oligomer is produced by a solid phase reaction, for example, a monomer including a base at a 3' end of the nucleic acid oligomer can bind to a solid-phase support via an oxygen atom bound to a carbon atom at position 3' in a ribose structure. In this case, there may be a linker between the monomer and the solid-phase support, if necessary.

A type of the solid-phase support is not particularly limited. Examples thereof include controlled-pore glass, oxalylated controlled-pore glass, a TentaGel support-aminopolyethylene glycol derivatized support, highly crosslinked aminomethylpolystyrene, a Pros-polystyrene/divinylbenzene copolymer, etc. The solid-phase support and the monomer may be linked using an amino group on the solid-phase support. Examples of the amino group on the solid-phase support include a 3-aminopropyl group, a long alkylamino group (LCAA), etc. There may be a linker between the solid-phase support and the monomer. Examples of the linker include a succinyl group, an oxalyl group, etc.

In the oxidizing step, the precursor nucleic acid oligomer obtained in the condensing step is oxidized with an oxidizing agent to obtain the nucleic acid oligomer according to the present embodiment. Examples of the oxidizing agent include a combination of (+)-camphorylsulfonyloxaziridine (CSO), (+)-(8,8-dichlorocamphorylsulfonyl)-oxaziridine (DCSO), t-butyl hydroperoxide, methyl ethyl ketone peroxide, a positive halogen reagent (carbon tetrachloride, carbon tetrabromide, iodine, N-chlorosuccinimide, N-bromosuccinimide, N-iodosuccinimide, etc.) with water, etc. The oxidizing step may be performed in the presence of a basic compound serving as the oxidizing agent. Examples of the basic compound include triethylamine, diisopropylethylamine, etc.

The condensing step in the method for producing a nucleic acid oligomer according to the present embodiment includes a condensing reaction of a hydroxy group bound to a carbon atom at position 5' in a ribose structure in a nucleotide structure of the nucleic acid oligomer with a phosphate moiety of the monomer. Therefore, when the hydroxy group bound to a carbon atom at position 5' has a protecting group, for example, a step of reacting the protecting group with a deprotecting reagent to eliminate the protecting group (first protecting group elimination step) is included. The first protecting group elimination step may be performed at any time as long as the condensing reaction in the method for producing a nucleic acid oligomer proceeds well, but is preferably performed before the condensing reaction. The first protecting group elimination step may use a deprotecting agent. Examples of the deprotecting agent that may be used in the first protecting group elimination step include a halogenated alkylcarboxylic acid, etc. Examples of the halogenated alkylcarboxylic acid include trifluoroacetic acid, trichloroacetic acid, dichloroacetic acid, etc. When a trityl-based protecting group such as triphenylmethyl group, a 4,4'-dimethoxytrityl (DMTr) group, or a 4-methoxytrityl (MMTr) group is used as the protecting group that the hydroxy group bound to a carbon atom at position 5' has, a cation scavenger is desirably added in order to avoid a side reaction of a borano group with a trityl cation produced in the first protecting group elimination step. Examples of the cation scavenger include triethylsilane. The nucleic acid oligomer having the protecting group is preferably reacted with 2 to 100 equivalents of the deprotecting agent.

Furthermore, when a base in the monomer to be used for the method for producing a nucleic acid oligomer has a protecting group, the method may further include a step of eliminating the protecting group of the base (second protecting group elimination step). The second protecting group elimination step may be performed at any time as long as a base in the eventually resulting nucleic acid oligomer does not have the protecting group. In the second protecting group elimination step, for example, the monomer or the nucleic acid oligomer is treated with ammonia water. Examples of the ammonia water to be used in the second protecting group elimination step include 25% by mass ammonia water or a 25% by mass ammonia water-ethanol mixed solution (3:1, v/v).

The resulting nucleic acid oligomer may be purified by a known purification method, for example, reverse-phase high-performance liquid chromatography (reverse-phase HPLC), ion exchange HPLC, column chromatography, recrystallization, etc.

### EXAMPLES

Hereinafter, the present invention will be more specifically described with reference to Examples, but the present invention is not limited to the Examples. Analyzers described below were used:
¹H-nuclear magnetic resonance spectrum (¹H-NMR): JEOL JNM-ECZ400S (400MHz)
³¹P-nuclear magnetic resonance spectrum (³¹P-NMR): JEOL JNM-ECZ400S (162MHz)

Note that, tetramethylsilane (TMS) was used as an internal standard for 1H-NMR and 85% H₃PO₄ was used as an external standard for ³¹P-NMR.

HRMS (ESI-TOF): SciexX500R QTOF
Ion exchange HPLC: GE Healthcare AKTA purifier 10S

As silica gel to be filled in column chromatography, Silica Gel 60N from KANTO CHEMICAL was used.
Column used for reverse-phase HPLC (analytical): waters µ-BOUNDASPHERE, C18 5 µm, 100 Å, 3.9 mm x 159 mm
Column used for ion exchange HPLC (analytical and preparative): GE Healthcare MiniQTM 4.6/50PE, 3 µm, 4.6 mm x 50 mm)
Reverse-phase UHPLC: waters ACQUITY UPLC H-Class Bio
PDA: waters ACQUITY UPLC PDA eλ Detector
LRMS(ESI): waters ACQUITY QDa Detector
Column used for reverse-phase UHPLC (analytical): waters ACQUITY UPLC Oligonucleotide BEH C18 Column, 130 Å, 1.7 um, 2.1 mm x 100 mm

Note that, unless otherwise indicated, "%" is by mass. In Examples, DMTr means 4,4'-dimethoxytrityl and other abbreviations have the same definition as described above.

### <Monomer Synthesis 1>

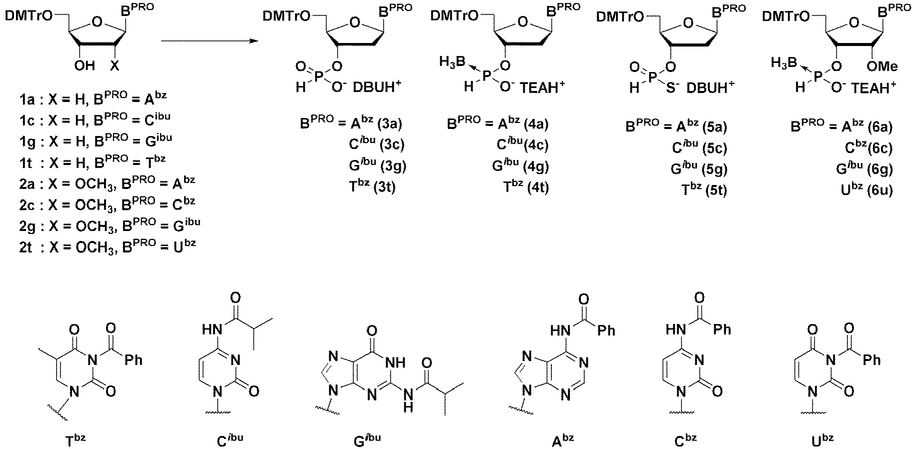

### (Synthesis of H-phosphonate monomer and H-boranophosphonate monomer)

H-phosphonate monomers 3a, 3c, 3g, and 3t and H-boranophosphonate monomers 4a, 4c, 4g, and 4t were synthesized by the known method (J. Org. Chem., 2019, Vol. 84, pp. 15032-15041; J. Org. Chem., 2014, Vol. 79, pp. 3465-3472).

### (Synthesis of H-phosphonothioate monomer)

H-phosphonothioate was synthesized with reference to the known method (J. Org. Chem., 1990, Vol. 55, pp. 3503-3506) according to the below-described scheme.

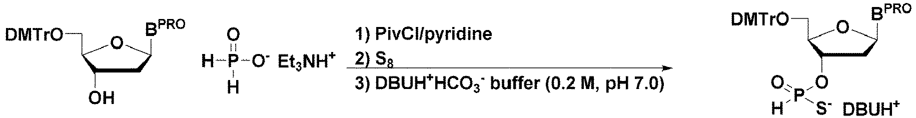

A nucleoside derivative 1a, 1c, 1g, or 1t shown on the far left in the scheme above (A: 0.90 g, 1.5 mmol; C: 0.96 g, 1.5 mmol; G: 0.97 g, 1.5 mmol; or T: 0.96 g, 1.5 mmol) and 0.19 mL (1.0 mmol) of triethylammnonium phosphinate were azeotropically dried with pyridine (3 mL x 3) under an argon atmosphere to be dissolved in pyridine (5 mL). Then, 0.17 mL (1.5 mmol) of pivaloyl chloride was added to the resulting pyridine solution with stirring in an ice bath (0°C) and stirred for 15 min. Thereafter, a reaction temperature was increased to room temperature (25°C) and 48 mg (1.5 mmol) of sulfur was added thereto and stirred for another 1 hour. The resulting solution was diluted with 30 mL of chloroform and then washed by adding 1M TEAB buffer solution (pH 7.0, 30 mL x 3). The resulting aqueous layer was back-extracted with chloroform (30 mL x 3). The resulting organic layer was dehydrated over anhydrous sodium sulfate. Then, the solvent in the organic layer was distilled off, the resulting residue was separated and purified by silica gel column chromatography (eluent: A: ethyl acetate-methanol-triethylamine (100:0:1 to 100:4:1, v/v/v) and then chloroform-methanol-triethylamine (100:2:1, v/v/v); C: ethyl acetate-methanol-triethylamine (100:0:1 to 100:4:1, v/v/v) and then chloroform-methanol-triethylamine (100:4:1, v/v/v); G: ethyl acetate-methanol-triethylamine (100:2:1, v/v/v) and then chloroform-methanol-triethylamine (100:3:1 to 100:5:1, v/v/v); T: ethyl acetate-methanol-triethylamine (100:0:1 to 100:1:1, v/v/v) and then chloroform-methanol-triethylamine (100:0:1 to 100:3:1, v/v/v)) and subjected to salt exchange with 0.2 M DBU bicarbonate. The organic layer was dried over anhydrous sodium sulfate and the solvent was distilled off under reduced pressure to thereby obtain a compound of interest (H-phosphonothioate monomer 5a, 5c, 5g, or 5t). Yield A: 0.88 g, 0.95 mmol, 95%; C: 0.89 g, 0.99 mmol, 99%; G: 0.58 g, 0.64 mmol, 64%; T: 0.64g, 0.65 mmol, 65%.

### H-phosphonothioate monomer 5a

¹H-NMR (400MHz, CDCl₃) δ 11.9-11.7 (br, 1H), 9.2-8.9 (br, 1H), 8.71 (s, 1H), 8.20 (s, 1H), 8.17 (d, J=574Hz, 0.5H), 8.13 (d, J=574Hz, 0.5H), 8.01 (d, J=7.3Hz, 2H), 7.62-7.57 (m, 1H), 7.54-7.48 (m, 2H), 7.46-7.38 (m, 3H), 7.34-7.10 (m, 6H), 6.79 (d, J=8.7Hz, 4H), 6.65-6.57 (m, 1H), 5.43-5.35 (m, 1H), 4.57-4.54 (m, 0.5H), 4.48-4.44 (m, 0.5H), 3.76 (s, 6H), 3.52-3.36 (m, 8H), 2.98-2.84 (m, 4H), 1.99 (quintet, J=5.8Hz, 2H), 1.80-1.60 (m, 6H).

³¹P-NMR (162MHz, CDCl₃) δ 53.0, 52.9.

### H-phosphonothioate monomer 5c

¹H-NMR (400MHz, CDCl₃) δ 11.7-11.4 (br, 1H), 8.8-8.5 (br, 1H), 8.19 (d, J=5.0Hz, 0.5H), 8.17 (d, J=5.0Hz, 0.5H), 8.13 (d, J=574Hz, 0.5H), 8.10 (d, J=581Hz, 0.5H), 7.46-7.39 (m, 2H), 7.13 (d, J=6.0Hz, 0.5H), 7.11 (d, J=6.0Hz, 0.5H), 6.90-6.81 (m, 4H), 6.34-6.24 (m, 1H), 6.30 (t, J=6.0Hz, 0.5H), 6.29 (t, J=6.0Hz, 0.5H)5.37-5.29 (m, 0.5H), 5.25-5.17 (m, 0.5H), 4.48 (q, J=3.0Hz, 0.5H), 4.36 (q, J=3.3Hz, 0.5H), 3.80 (s, 6H), 3.54-3.29 (m, 8H), 2.92-2.82 (m, 3H), 2.69-2.61 (m, 1H), 2.38-2.26 (m, 1H), 2.00 (q, J=5.8Hz, 2H), 1.82-1.60 (m, 7H), 1.25-1.17 (m, 6H).

³¹P-NMR (162MHz, CDCl₃) δ 53.6, 52.6.

### H-phosphonothioate monomer 5g

¹H-NMR (400MHz, CDCl₃) δ 8.12 (d, J=581Hz, 0.5H), 8.05 (d, J=574Hz, 0.5H), 7.82 (s, 0.5H), 7.80 (s, 0.5H), 7.44-7.31 (m, 3H), 7.31-7.22 (m, 4H), 7.22-7.13 (m, 2H), 6.79-6.67 (m, 4H), 6.18 (t, J=6.0Hz, 1H), 6.18 (t, J=6.4Hz, 1H), 5.75-5.62 (m, 0.5H), 5.62-5.51 (m, 0.5H), 4.37 (q, J=3.7Hz, 0.5H), 4.26 (q, J=3.7Hz, 0.5H), 3.75 (s, 6H), 3.50-3.25 (m, 7H), 3.02-2.83 (m, 1H), 2.83-2.74 (m, 2H), 2.74-2.58 (m, 2H), 2.03-1.87 (m, 2H), 1.83-1.53 (m, 6H), 1.20-1.07 (m, 6H).

³¹P-NMR (162MHz, CDCl₃) δ 52.8, 52.5.

### H-phosphonothioate monomer 5t

¹H-NMR (400MHz, CDCl₃) δ 11.6-11.5 (br, 1H), 8.13 (d, J=572Hz, 0.5H), 8.08 (d, J=578Hz, 0.5H), 7.92 (d, J=8.2Hz, 2H), 7.78 (s, 0.5H), 7.77 (s, 0.5H), 7.64 (t, J=7.3Hz, 1H), 7.53-7.39 (m, 5H), 7.41 (d, J=572Hz, 0.5H), 7.36 (d, J=578Hz, 0.5H), 7.38-7.27 (m, 5H), 7.26-7.18 (m, 1H), 6.91-6.82 (m, 4H), 6.44 (t, J=8.7Hz, 0.5H), 6.43 (t, J=8.9Hz, 0.5H), 5.51-5.41 (m, 0.5H), 5.41-5.32 (m, 0.5H), 4.41 (d, J=1.4Hz, 0.5H), 4.30 (d, J=2.3Hz, 0.5H), 3.79 (s, 6H), 3.63-3.46 (m, 1H), 3.45-3.31 (m, 1H), 2.87-2.62 (m, 3H), 2.54-2.38 (m, 1H), 1.93 (q, J=5.8Hz, 2H), 1.78-1.54 (m, 6H), 1.37 (d, J=6.9Hz, 3H).

³¹P-NMR (162MHz, CDCl₃) δ 54.5, 53.4.

### (Synthesis of 2'-OMe-modified H-boranophosphonate monomer)

According to the scheme below, a 2'-OMe-modified H-boranophosphonate monomer was synthesized.

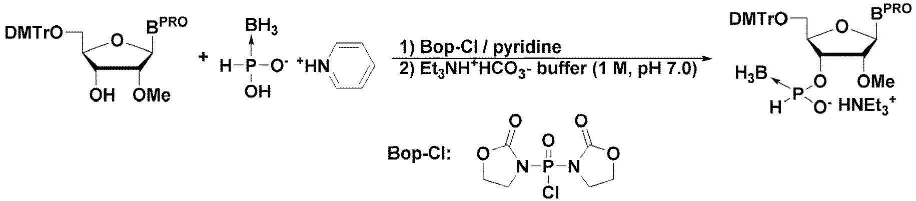

A nucleoside derivative 2a, 2c, 2g, or 2t shown on the far left in the scheme above (A: 0.69 g, 1.0 mmol; C: 0.68 g, 1.0 mmol; G: 0.70 g, 1.0 mmol; or U: 0.67 g, 1 mmol) and pyridinium H-boranophosphonate (0.28 g, 2.0 mmol) were azeotropically dried with pyridine (3 mL x 3) under an argon atmosphere to be dissolved in 25 mL of pyridine. BopCl (0.51 g, 2.0 mmol) was added to the resulting pyridine solution with stirring in an ice bath. Then, the reaction temperature was increased to room temperature and stirring was continued for 1 hour. The resulting solution was diluted with 30 mL of chloroform and then the resulting organic layer was washed with 1.0 M TEAB buffer solution (pH 7.0) (30 mL x 3). The resulting aqueous layer was back-extracted with chloroform (30 mL x 3). The organic layer was collected and dehydrated over anhydrous sodium sulfate, and then, the solvent was distilled off from the organic layer under reduced pressure. The resulting residue was separated and purified by silica gel column chromatography (eluent: A: ethyl acetate-methanol-triethylamine (100:0:1 to 100:4:1, v/v/v) and then chloroform-methanol-triethylamine (100:4:1, v/v/v); C: ethyl acetate-methanol-triethylamine (100:0:1 to 100:4:1, v/v/v) and then chloroform-methanol-triethylamine (100:4:1, v/v/v); G: ethyl acetate-methanol-triethylamine (100:2:1, v/v/v) and then chloroform-methanol-triethylamine (100:3:1 to 100:5:1, v/v/v); U: ethyl acetate-methanol-triethylamine (100:0:1, v/v/v) and then chloroform-methanol-triethylamine (100:0:1 to 100:1:1, v/v/v)) and extracted with 1.0 M TEAB buffer solution (pH 7.0) and chloroform (30 mL x 3). The organic layer was dehydrated over anhydrous sodium sulfate and the solvent was distilled off under reduced pressure. Thus, a product of interest (2'-OMe-modified H-boranophosphonate monomer 6a, 6c, 6g, or 6u) was obtained. Yield A: 0.81 g, 0.95 mmol, 95%; C: 0.75 g, 0.90 mmol, 90%; G: 0.78 g, 0.92 mmol, 92%; U: 0.62 g, 0.74 mmol, 74%

### 2'-OMe-modified H-boranophosphonate monomer 6a

¹H-NMR (400MHz, CDCl₃) δ 9.09 (s, 1H), 8.70 (s, 0.5H), 8.69 (s, 0.5H), 8.24 (s, 0.5H), 8.20 (s, 0.5H), 8.02 (d, J=7.8Hz, 2H), 7.9-7.8 (br, 0.5H), 7.60 (t, J=7.3Hz, 1H), 7.51 (t, J=7.8Hz, 2H), 7.48-7.42 (m, 2H), 7.36-7.31 (m, 4H), 7.30-7.23 (m, 2H), 7.23-7.17 (m, 1H), 6.84-6.78 (m, 4H), 6.27 (t, J=5.0Hz, 0.5H), 6.27 (t, J=5.0Hz, 0.5H), 5.09-5.02 (m, 1H), 4.74-4.68 (m, 1H), 4.55-4.50 (m, 1H), 3.77 (s, 6H), 3.55-3.48 (m, 5H), 2.99 (q, J=7.3Hz, 6H), 1.27 (t, J=7.3Hz, 9H), 0.9-0.1 (br, 3H).

³¹P-NMR (162MHz, CDCl₃) δ 109-104 (br).

### 2'-OMe-modified H-boranophosphonate monomer 6c

¹H-NMR (400MHz, CDCl₃) δ 8.65 (d, J=7.8Hz, 0.5H), 8.59 (d, J=7.3Hz, 0.5H), 7.95-7.86 (m, 2H), 7.9-7.8 (br, 0.5H), 7.60 (t, J=6.9Hz, 1H), 7.51 (t, J=7.8Hz, 2H), 7.44 (dd, J=7.1, 5.7Hz, 2H), 7.39-7.24 (m, 7H), 7.2-7.0 (br, 0.5H), 6.92-6.85 (m, 4H), 6.05 (s, 0.5H), 6.02 (s, 0.5H), 5.05-4.92 (m, 0.5H), 4.82-4.75 (m, 0.5H), 4.40 (dd, J=2.1, 8.9Hz, 1H), 4.12-4.03 (m, 1H), 3.83 (s, 6H), 3.67 (s, 3H), 3.63-3.53 (m, 2H), 3.01 (q, J=7.2Hz, 6H), 1.27 (t, J=7.3Hz, 9H), 0.9-0.1 (br, 3H).

³¹P-NMR (162MHz, CDCl₃) δ 112-102 (br).

### 2'-OMe-modified H-boranophosphonate monomer 6g

¹H-NMR (400MHz, CDCl₃) δ 12.5-11.6 (br, 1H), 10.5-9.7 (br, 1H), 7.88 (s, 0.5H), 7.87 (s, 0.5H), 7.8-7.7 (br, 0.5H), 7.53-7.36 (m, 2H), 7.34-7.28 (m, 4H), 7.26-7.13 (m, 3H), 6.93-6.83 (br, 0.5H), 6.83-6.66 (m, 4H), 5.93 (d, J=4.6Hz, 0.5H), 5.90 (d, J=4.6Hz, 0.5H), 5.37-5.29 (m, 0.5H), 5.29-5.20 (m, 0.5H), 4.58 (t, J=4.8Hz, 0.5H), 4.56 (t, J=4.8Hz, 0.5H), 4.44-4.36 (m, 0.5H), 4.36-4.28 (m, 0.5H), 3.75, 3.75, 3.74 (s, s, s, 6H), 3.54-3.37 (m, 4H), 3.37-3.22 (m, 1H), 3.00 (q, J=7.2Hz, 6H), 2.62-2.40 (m, 1H), 1.25 (t, J=7.3Hz, 9H), 1.11 (d, J=6.9Hz, 3H), 1.04 (d, J=5.5Hz, 3H), 1.0-0.1 (br, 3H).

³¹P-NMR (162MHz, CDCl₃) δ 108-103 (br).

### 2'-OMe-modified H-boranophosphonate monomer 6u

¹H-NMR (400MHz, CDCl₃) δ 8.23 (d, J=8.2Hz, 0.5H), 8.17 (d, J=8.2Hz, 0.5H), 8.01-7.90 (m, 2H), 7.65 (t, J=7.3Hz, 1H), 7.51 (t, J=7.8Hz, 2H), 7.47-7.38 (m, 2H), 7.38-7.21 (m, 7H), 6.87 (m, 4H), 5.97 (d, J=2.3Hz, 0.5H), 5.97 (d, J=2.1Hz, 0.5H), 5.30 (d, J=2.7Hz, 0.5H), 5.27 (d, J=2.7Hz, 0.5H), 5.10-5.03 (m, 0.5H), 4.95-4.86 (m, 0.5H), 4.38-4.33 (m, 1H), 4.08-4.05 (m, 1H), 3.80 (s, 6H), 3.67-3.58 (m, 1H), 3.55 (d, J=4.6Hz, 4H), 3.10 (q, J=7.2Hz, 6H), 1.28 (t, J=7.2Hz, 9H), 1.0-0.2 (m, 3H) .

³¹P-NMR (162MHz, CDCl₃) δ 113-103 (br).

### <Synthesis of nucleic acid oligomer by solid-phase method 1>

Hereinafter, among internucleotide bonds, a phosphorothioate bond is indicated by a subscript PS, a boranophosphate bond is indicated by a subscript PB, and a phosphodiester bond is indicated by a subscript PO.

### (Solid-phase synthesis of T_{PS}T)

### [Comparative Synthesis Example 1]

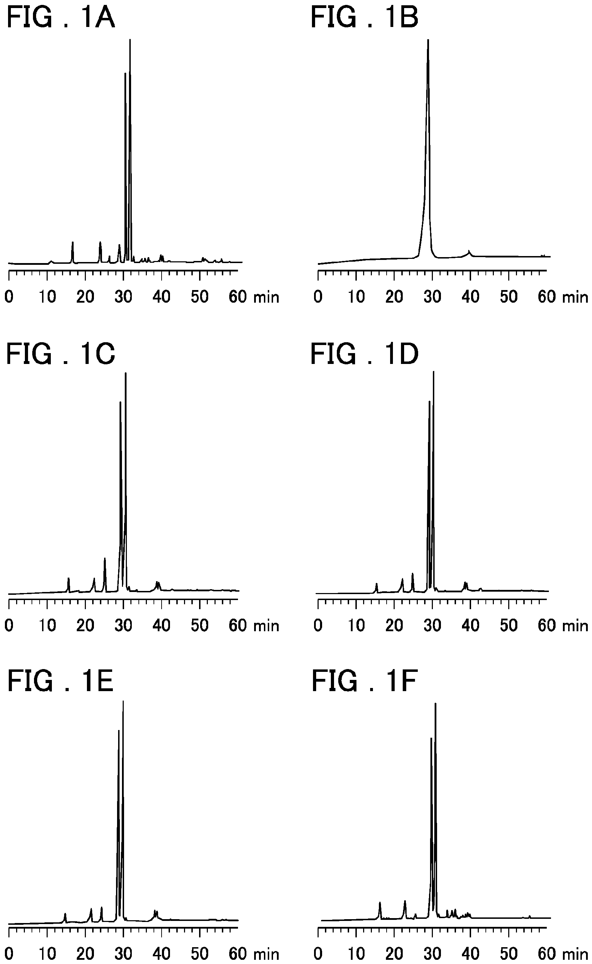

First, 5'-dimethoxytrityl-N³-benzoylthymidine (0.5 µmol) supported on a solid-phase support via a succinyl linker was detritylated with the addition of a 3% dichloroacetic acid/dichloromethane solution (5 x 12s, each 1 mL) and then the solid-phase support was washed with acetonitrile and dichloromethane dried by molecular sieve. The solid-phase support was dried for 10 min and 19.7 mg (40 equivalents, 20 µmol) of H-phosphonothioate monomer 5t and bis(2,6-dimethylphenyl)phosphorochloridate (hereinafter referred to as "(DMP)₂CP") (16.2 mg, 100 equivalents, 50 µmol) serving as a condensing agent according to the non-patent Document (Tetrahedron Lett. 2004, 45, 5803-5806.) were added to a reaction system. An acetonitrile solution containing pyridine serving as a base (pyridine: acetonitrile = 1:4, v/v) in the presence of argon was added as a reaction solvent and manually stirred slowly for 3 min. Thus, the monomer was condensed. After the H-phosphonothioate monomer was condensed, the solid-phase support was washed with acetonitrile (1 mL x 4) and dichloromethane (1 mL x 4) dried as described above and then a dimethoxytrityl group was deprotected with a 3% dichloroacetic acid/dichloromethane-triethylsilane (1:1, v/v) solution (4 x 15 s, each 1 mL). Then, the solid-phase support was washed with acetonitrile (1 mL x 4) and dichloromethane (1 mL x 4) dried as described above and dried for 10 min. The resulting H-phosphonothioate bond was oxidized for 90 min with the addition of a 0.1 M triethylamine/carbon tetrachloride-2,6-lutidine-water (5:12.5:1, v/v/v) solution to thereby be converted to a phosphorothioate bond. The solid-phase support was washed with acetonitrile (1 mL x 4) and dichloromethane (1 mL x 4) dried as described above and reacted with a 25% ammonia water/ethanol (3:1, v/v, 5 mL) solution at room temperature for 3 hours. Thus, a base moiety of a nucleic acid was deprotected and a nucleic acid oligomer was cut from the solid-phase support. After the reaction, the reaction solution was filtered and washed with ethanol. The solvent was distilled off under reduced pressure and the resulting crude product was analyzed by reverse-phase HPLC. The reverse-phase HPLC was performed in a 0.1 M TEAA buffer solution (pH 7) using a linear concentration gradient from 0 to 30% acetonitrile at 30°C for 60 min at a flow rate of 0.5 mL/min. Results are shown in Table 1 and FIG. 1A.

### [Comparative Synthesis Example 2] Control experiment of Comparative Synthesis Example 1

Experimental procedures were performed in the same manner as in Comparative Synthesis Example 1, except that (DMP)₂CP was added instead of the H-phosphonothioate monomer 5t. Results are shown in FIG. 1B.

### [Synthesis Example 1]

Procedures were performed in the same manner as in Comparative Synthesis Example 1, except that BOMP (23.3 mg, 100 equivalents, 50 µmol) was used as the condensing agent instead of (DMP)₂CP and a solution of 2,6-lutidine (base, 0.6 M, 120 µmol) in acetonitrile was used as the reaction solvent. Results are shown in Table 1.

### [Synthesis Example 2]

Procedures were performed in the same manner as in Synthesis Example 1, except that MNTP (22.3 mg, 100 equivalents, 50 µmol) was used as the condensing agent instead of BOMP. Results are shown in Table 1 and FIG. 1C.

### [Synthesis Example 3]

Procedures were performed in the same manner as in Synthesis Example 1, except that PyNTP (25.0 mg, 100 equivalents, 50 µmol) was used as the condensing agent instead of BOMP. Results are shown in Table 1 and FIG. 1D.

### [Synthesis Example 4]

Procedures were performed in the same manner as in Synthesis Example 3, except that pyridine (0.6 M, 120 µmol) was used as the base added upon the condensing reaction instead of 2,6-lutidine. Results are shown in Table 1.

### [Synthesis Example 5]

Procedures were performed in the same manner as in Synthesis Example 3, except that quinoline (0.6 M, 120 µmol) was used as the base added upon the condensing reaction instead of 2,6-lutidine. Results are shown in Table 1.

### [Synthesis Example 6]

Procedures were performed in the same manner as in Synthesis Example 3, except that quinoline (1.8 M, 355 µmol) was used as the base added upon the condensing reaction instead of 2,6-lutidine. Results are shown in Table 1 and FIG. 1E.

### [Synthesis Example 7]

Procedures were performed in the same manner as in Synthesis Example 3, except that only acetonitrile was used as the reaction solvent, that is, the base was not added upon the condensing reaction. Results are shown in Table 1 and FIG. 1F.

**[Table 1]**

| | Condensing condition | | Chemoselectivity (PO:PS) | HPLC yield (%) |
|---|---|---|---|---|
| | Condensing agent | Base | | |
| Comparative Synthesis Example 1 | (DMP) ₂CP | Pyridine-acetonitrile (1:4, v/v) | 1:99 | 94 |
| Synthesis Example 1 | BOMP | 2,6-Lutidine | - | <1 |
| Synthesis Example 2 | MNTP | 2,6-Lutidine | 6:94 | 91 |
| Synthesis Example 3 | PyNTP | 2,6-Lutidine | 4:96 | 94 |
| Synthesis Example 4 | PyNTP | Pyridine | 3:97 | 94 |
| Synthesis Example 5 | PyNTP | Quinoline (0.6 M) | 2:98 | 94 |
| Synthesis Example 6 | PyNTP | Quinoline (1.8 M) | 3:97 | 95 |
| Synthesis Example 7 | PyNTP | - | 1:99 | 97 |

In Table 1, chemoselectivity was calculated from an area ratio of a byproduct T_{PO}T to a product of interest T_{PS}T based on the HPLC results. An HPLC yield was calculated from an area ratio T_{PS}T / (T + T_{PO}T + T_{PS}T) based on the HPLC results.

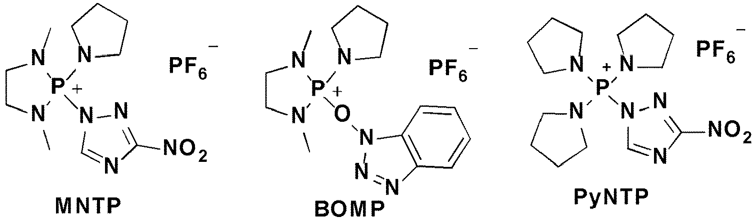

FIGs. 1A to 1F show reverse-phase HPLC analysis results of Comparative Synthesis Examples 1 and 2 and Synthesis Examples 2, 3, 6, and 7. Compared to Comparative Synthesis Example 1, Synthesis Examples 6 and 7 resulted in a higher yield of the product of interest. Note that, an unidentified peak was observed around an elution time of 28 min only in Comparative Synthesis Example 1. Then, the control experiment in which no monomer was added upon the condensation reaction was performed in Comparative Synthesis Example 2, and a peak was observed at an elution time of 28 min by reversed-phase HPLC. Therefore, the peak observed around the elution time of 28 min in Comparative Synthesis Example 1 is considered to be a byproduct resulting from a reaction of a hydroxyl group with the condensing agent. Dimers were synthesized in Synthesis Examples and Comparative Synthesis Examples above, but it is feared that when such a side reaction proceeds during synthesis of an oligomer, a reaction efficiency is greatly reduced. Therefore, the methods in Synthesis Examples were confirmed to be suitable for synthesis of the oligomer.

### (Solid-phase synthesis of N_{PS}T)

### [Synthesis Example 8]

First, 5'-dimethoxytrityl-N³-benzoylthymidine (0.5 µmol) supported on a solid-phase support via a succinyl linker was detritylated with the addition of a 3% dichloroacetic acid/dichloromethane solution (4 x 15 s, each 1 mL) and then the solid-phase support was washed with acetonitrile and dichloromethane dried by molecular sieve. The solid-phase support was dried for 10 min and H-phosphonothioate monomer 5a, 5c, or 5g (A: 19.0 mg (40 equivalents, 20 µmol); C: 17.8 mg (40 equivalents, 20 µmol); or G: 18.1 mg (40 equivalents, 20 µmol)) and PyNTP (100 equivalents, 50 µmol) were added to a reaction system. An acetonitrile solution containing quinoline (1.8 M) serving as a base in the presence of argon was added as a reaction solvent and manually stirred slowly for 3 min. Thus, the monomer was condensed. After the H-phosphonothioate monomer was condensed, the solid-phase support was washed with acetonitrile (1 mL x 4) and dichloromethane (1 mL x 4) dried as described above and then a dimethoxytrityl group was deprotected with a 3% dichloroacetic acid/dichloromethane-triethylsilane (1:1, v/v) solution (4 x 15 s, each 1 mL). Then, the solid-phase support was washed with acetonitrile (1 mL x 4) and dichloromethane (1 mL x 4) dried as described above and dried for 10 min. The thus-formed H-phosphonothioate bond was oxidized for 90 min with the addition of a 0.1 M triethylamine/carbon tetrachloride-2,6-lutidine-water (5:12.5:1, v/v/v) solution to thereby be converted to a phosphorothioate bond. The solid-phase support was washed with acetonitrile (1 mL x 4) and dichloromethane (1 mL x 4) dried as described above and reacted with a 25% ammonia water/ethanol (3:1, v/v, 5 mL) solution under the below-described conditions. Thus, a base moiety of a nucleic acid was deprotected and a nucleic acid oligomer was cut from the solid-phase support (A_{PB}T and C_{PB}T: room temperature, 20h; G_{PB}T: 50°C, 20h). After the reaction, the reaction solution was filtered and washed with ethanol. The solvent was distilled off under reduced pressure and the resulting crude product was analyzed by reverse-phase HPLC in the same manner as above. Results are shown in Table 2.

### [Synthesis Example 9]

Procedures were performed in the same manner as in Synthesis Example 8, except that only acetonitrile was used as the reaction solvent, that is, the base was not added. Results are shown in Table 2.

### [Synthesis Example 10]

Synthesis Example 10 was performed only when H-phosphonothioate monomer 5g was used. Procedures were performed in the same manner as in Synthesis Example 9, except that the amount of the monomer was changed to 36.2 mg (80 equivalents, 40 µmol) and the amount of the PyNTP was changed to 50.0 mg (200 equivalents, 100 µmol). Results are shown in Table 2.

**[Table 2]**

| Entry | Condensing condition | | | Chemoselectivity (PO:PS) | HPLC yield (%) |
|---|---|---|---|---|---|
| | Monomer | Base | Product | | |
| 1 | 5a | Quinoline | dA_{PS}T | 2:98 | 95 |
| 2 | 5a | - | dA_{PS}T | 3:97 | 70 |
| 3 | 5c | Quinoline | dC_{PS}T | 2:98 | 96 |
| 4 | 5c | - | dC_{PS}T | 2:98 | 94 |
| 5 | 5g | Quinoline | dG_{PS}T | 0:100 | 88 |
| 6 | 5g | - | dA_{PS}T | 0:100 | 91 |
| 7 | 5g | - | dA_{PS}T | 0:100 | 93 |

In Table 2, chemoselectivity was calculated from an area ratio of a byproduct dN_{PO}T to a product of interest dN_{PS}T based on the HPLC results. A HPLC yield was calculated from an area ratio dT_{PS}T / (T + dT_{PO}T + dT_{PS}T) based on the HPLC results. The monomer was 40 equivalents and PyNTP was 100 equivalents for Entries 1 to 6, whereas the monomer was 80 equivalents and PyNTP was 200 equivalents for Entry 7. Entries 1, 3, and 5 show results of Synthesis Example 8, Entries 2, 4, and 6 show results of Synthesis Example 9, and Entry 7 shows result of Synthesis Example 10.

### (Solid-phase synthesis of N*_{PB}T)

### [Synthesis Example 11]

Hereinafter, N* denotes a 2'-OMe-modified nucleotide unit. First, 5'-dimethoxytrityl-N³-benzoylthymidine (0.5 µmol) supported on a solid-phase support via a succinyl linker was detritylated with the addition of a 3% dichloroacetic acid/dichloromethane solution (4 x 15 s, each 1 mL) and then the solid-phase support was washed with acetonitrile and dichloromethane dried by molecular sieve. The solid-phase support was dried for 10 min and 2'-OMe-modified H-boranophosphonate monomer 6a, 6c, 6g, or 6u (A: 17.1 mg (40 equivalents, 20 µmol); C: 16.7 mg (40 equivalents, 20 µmol); G: 16.9 mg (40 equivalents, 20 µmol); U: 16.7 mg (40 equivalents, 20 µmol)) and MNTP (22.3 mg, 100 equivalents, 50 µmol) were added to a reaction system. An acetonitrile solution containing 2,6-lutidine serving as a base in the presence of argon was added as a reaction solvent and manually stirred slowly for 3 min. Thus, the monomer was condensed. After the 2'-OMe-modified H-boranophosphonate monomer was condensed, the solid-phase support was washed with acetonitrile (1 mL x 4) and dichloromethane (1 mL x 4) dried as described above and then a dimethoxytrityl group was deprotected with a 3% dichloroacetic acid/dichloromethane-triethylsilane (1:1, v/v) solution (4 x 15 s, each 1 mL). Then, the solid-phase support was washed with acetonitrile (1 mL x 4) and dichloromethane (1 mL x 4) dried as described above and dried for 10 min. The thus-formed H-boranophosphonate bond was oxidized for 90 min with the addition of a 0.1 M triethylamine/carbon tetrachloride-2,6-lutidine-water (5:12.5:1, v/v/v) solution to thereby be converted to a boranophosphate bond. The solid-phase support was washed with acetonitrile (1 mL x 4) and dichloromethane (1 mL x 4) dried as described above and reacted with a 25% ammonia water/ethanol (3:1, v/v, 5 mL) solution under the below-described conditions. Thus, a base moiety of a nucleic acid was deprotected and a nucleic acid oligomer was cut from the solid-phase support (U*_{PB}T: room temperature, 3h; A*_{PB}T and C*_{PB}T: room temperature, 20h; G*_{PB}T: 50°C, 20h). After the reaction, the reaction solution was filtered and washed with ethanol. The solvent was distilled off under reduced pressure and the resulting crude product was analyzed by reverse-phase HPLC in the same manner as above. Results are shown in Table 3.

**[Table 3]**

| Entry | Monomer | Product | HPLC yield (%) |
|---|---|---|---|
| 1 | 6a | A*_{PB}T | 95 |
| 2 | 6c | C*_{PB}T | 96 |
| 3 | 6g | G*_{PB}T | 98 |
| 4 | 6u | U*_{PB}T | 97 |

In Table 3, a HPLC yield was calculated from an area ratio N*_{PB}T / (T + N*_{PB}T) based on the HPLC results.

### [Example 1] Synthesis of PB/PS/PO chimeric nucleic acid oligomer (dodecamer)

First, 5'-dimethoxytrityl-N³-benzoylthymidine (0.5 µmol) supported on a solid-phase support via a succinyl linker was reacted with a 3% dichloroacetic acid/dichloromethane solution (1 mL) for 15 seconds to eliminate a protecting group and then the resulting reaction solution was removed. The same procedures were repeated four times, followed by washing with dichloromethane (1 mL) four times and with acetonitrile (1 mL) four times, and the solid phase support was vacuum dried. Next, an oligomer chain elongation reaction was performed by repeating a combination of steps (i) and (ii) below eleven times.

Step (i): a monomer selected depending on a sequence, MNTP or PyNTP, and optionally 2,6-lutidine or quinoline were mixed in acetonitrile (0.2 mL) to react with each other for 3 min. Three minutes later, the resulting reaction solution was removed and the thus-reacted solid-phase support was washed with acetonitrile four times and with dichloromethane four times. Condensing conditions using various monomers were described below.

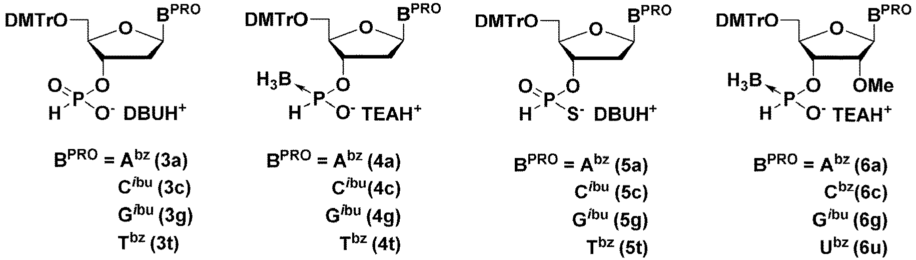

**[Table 4]**

| Monomer | | Condensing agent | | Base | |
|---|---|---|---|---|---|
| 3a, 3c, 3g, 3t | 40 equivalents, 20 µmol | MNTP | 100 equivalents, 50 µmol | 2,6-Lutidine | 0.6 M |
| 4a, 4c, 4g, 4t | 40 equivalents, 20 µmol | MNTP | 100 equivalents, 50 µmol | 2,6-Lutidine | 0.6 M |
| 5a, 5c | 40 equivalents, 20 µmol | PyNTP | 100 equivalents, 50 µmol | Quinoline | 1.8 M |
| 5g | 80 equivalents, 40 µmol | PyNTP | 200 equivalents, 100 µmol | - | |
| 5t | 40 equivalents, 20 µmol | PyNTP | 100 equivalents, 50 µmol | - | |
| 6a, 6c, 6g, 6t | 40 equivalents, 20 µmol | MNTP | 100 equivalents, 50 µmol | 2,6-Lutidine | 0.6 M |

Step (ii): the solid-phase support that has undergone Step (i) was reacted with a 3% dichloroacetic acid/dichloromethane-triethylsilane (1:1, v/v) solution (1 mL) for 15 seconds. The same procedures were repeated four times, followed by washing with dichloromethane (1 mL) four times and with acetonitrile (1 mL) four times, and the solid phase support was vacuum dried.

After the chain elongation, 0.5 mL of a 0.1 M triethylamine/carbon tetrachloride-2,6-lutidine-H₂O (5:12.5:1, v/v/v) solution was added to the solid-phase support to react with each other for 90 min. After the reaction, the solid-phase support was washed with acetonitrile (1 mL) four times and with dichloromethane (1 mL) four times.

Then, the thus-washed solid-phase support was reacted with 25% ammonia water-ethanol (3:1, v/v) at 50°C for 20 hours. Thus, a base moiety of a nucleic acid was deprotected and a nucleic acid oligomer was cut from the solid-phase support. The solid-phase support was filtered out, a filtrate was collected, and then a solvent was distilled off under reduced pressure.

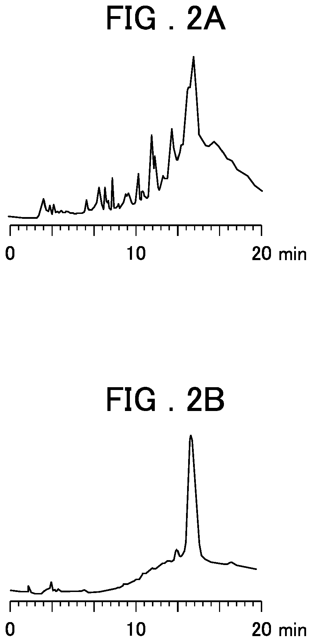

A solution containing the resulting PB/PS/PO chimeric nucleic acid oligomer (dodecamer) was treated with a 25% NH₃-ethanol aqueous solution (3:1, v/v, 50°C, 20h) and washed with ethanol (2 mL x 4). A solvent was distilled off from the aqueous solution under reduced pressure. Then, separation and purification were performed by reverse-phase HPLC. The reverse-phase HPLC and the separation and purification were performed at 60°C using a 0.1 M hexafluoroisopropanol-0.008 M triethylamine buffer solution containing from 5 to 40% methanol as a mobile phase at a flow rate of 0.5 mL/min for 20 min. Charts of reverse-phase HPLC before and after the separation and purification for Example 1 are shown in FIG. 2A and FIG. 2B, respectively. Yield: 6%. HRMS (ESI-TOF): Calcd for [M-6H]⁶⁻, 615.7786; found, 615.7767

The resulting PB/PS/PO chimeric nucleic acid oligomer (dodecamer) was a nucleic acid oligomer having the base sequence d(C_{PS}A_{PS}G_{PS}T_{PS}C_{PB}A_{PB}G_{PB}T_{PB}C_{PO}A_{PO}G_{PO}T) (SEQ ID NO: 1) .

### [Example 2] Synthesis of PB/PS/PO chimeric nucleic acid oligomer (apo B sequence containing dodecamer)

A portion of mRNA coding for an apoB protein (apoprotein B-100: Nature, 2006, Vol. 441, pp 111-114) (3'-rCGU AAC CAU AAG-5': complementary chain RNA, SEQ ID NO: 2) was selected as a target nucleic acid. A PB/PS/PO chimeric nucleic acid oligomer having a base sequence complementary to that of the RNA was synthesized.

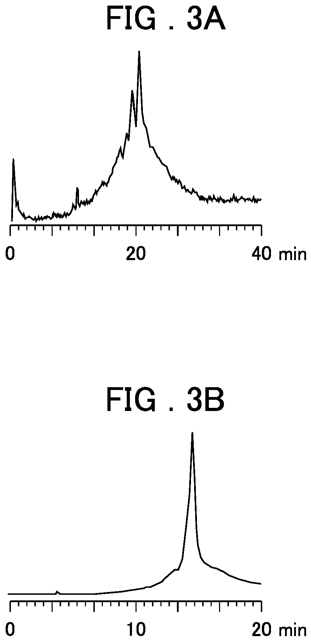

The above-described PB/PS/PO chimeric nucleic acid oligomer was synthesized by the same procedures as in Example 1 using 5'-O-DMTr-N⁴-benzoylcytidine (0.5 µmol) supported on a solid-phase support via a succinyl linker. The resulting nucleic acid oligomer was separated and purified by ion exchange HPLC. The separation and purification were performed at room temperature using 0 to 0.5M NaCl and a 10mM Tris-HCl buffer solution (pH7.5) containing 30% acetonitrile at a flow rate of 0.4 mL/min for 40 min. Charts of reverse-phase HPLC before and after the separation and purification for Example 2 are shown in FIG. 3A and FIG. 3B, respectively. Yield: 19%. HRMS (ESI-TOF) : Calcd for [M-6H]⁶⁻, 614.1138; found, 614.1136

The resulting PB/PS/PO chimeric nucleic acid oligomer (dodecamer) was an antisense molecule having the base sequence d(G_{PB}C_{PS}A_{PB}T_{PO}T_{PO}G_{PO}G_{PO}T_{PS}A_{PB}T_{PS}T_{PB}C) (SEQ ID NO: 3).

### [Example 3] Synthesis of PB/PS/PO-gapmer (dodecamer)

The monomer used in Example 2 and a 2'-OMe-modified boranophosphonate compound having N⁴-benzoylcytosine, N⁶-benzoyladenine, N²-isobutyrylguanine, or N³-benzoylthymine as a base were sequentially subjected to the deprotection reaction of a hydroxy group having a protecting group and the condensing reaction repeatedly until a dodecamer was obtained so as to give a base sequence complementary to a base sequence of the target nucleic acid (apoB protein) and then subjected to an oxidizing reaction and a deprotecting reaction of a base moiety of a nucleic acid, and a nucleic acid oligomer was cut from the solid-phase support in the same manner as in Example 2. Thus, a PB/PS/PO-gapmer (dodecamer) was obtained.

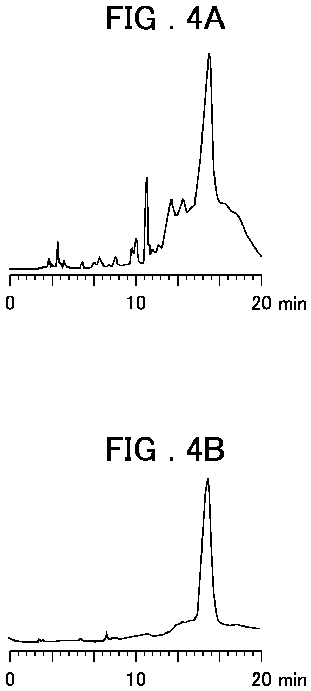

A solution containing the resulting PB/PS/PO-gapmer (dodecamer) was treated with a 25% NH₃-ethanol aqueous solution (3:1, v/v, 50°C, 20h) and washed with ethanol (2 mL x 4). A solvent was distilled off from the aqueous solution under reduced pressure. Then, separation and purification were performed by reverse-phase HPLC. The reverse-phase HPLC and the separation and purification were performed at 60°C using a 0.1 M hexafluoroisopropanol-0.008 M triethylamine buffer solution containing from 5 to 40% methanol as a mobile phase at a flow rate of 0.5 mL/min for 20 min. Charts of reverse-phase HPLC before and after the separation and purification for Example 3 are shown in FIG. 4A and FIG. 4B, respectively. Yield: 13%. HRMS (ESI-TOF) Calcd for [M-6H]⁶⁻, 635.9695; found, 635.9670

The resulting PB/PS/PO-gapmer (dodecamer) was an antisense molecule having the base sequence G*_{PB}C*_{PB}A*_{PB}d(T_{PS}T_{PO}G_{PO}G_{PO}T_{PS}A_{PB})U*_{PB}U*_{PB}C* (SEQ ID NO: 4).
(N* denotes a 2'-OMe-modified nucleotide unit).

### [Example 4] Synthesis of PB/PS chimeric nucleic acid oligomer (dodecamer)

The deprotection reaction of a hydroxy group having a protecting group and the condensing reaction were sequentially performed repeatedly until a dodecamer was obtained so as to give a base sequence complementary to a base sequence of the target nucleic acid (apoB protein) and then an oxidizing reaction and a deprotecting reaction of a base moiety of a nucleic acid were performed, and a nucleic acid oligomer was cut from the solid-phase support in the same manner as in Example 2. Thus, a PB/PS chimeric nucleic acid oligomer (dodecamer) was obtained.

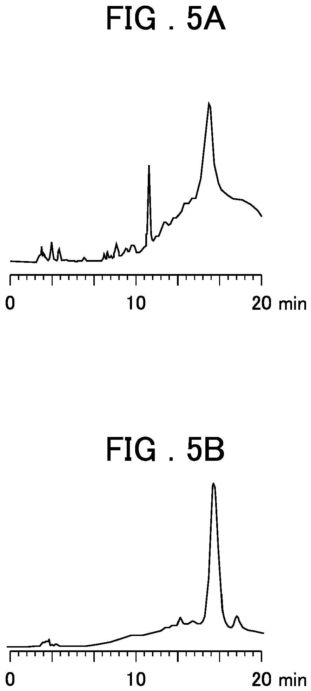

A solution containing the resulting PB/PS chimeric nucleic acid oligomer (dodecamer) was treated with a 25% NH₃-ethanol aqueous solution (3:1, v/v, 50°C, 20h) and washed with ethanol (2 mL x 4). A solvent was distilled off from the aqueous solution under reduced pressure. Then, separation and purification were performed by reverse-phase HPLC. The reverse-phase HPLC and the separation and purification were performed at 60°C using a 0.1 M hexafluoroisopropanol-0.008 M triethylamine buffer solution containing from 5 to 40% methanol as a mobile phase at a flow rate of 0.5 mL/min for 20 min. Charts of reverse-phase HPLC before and after the separation and purification for Example 4 are shown in FIG. 5A and FIG. 5B, respectively. Yield: 5%. HRMS (ESI-TOF) Calcd for [M-6H]⁶⁻, 618.6194; found, 618.6175

The resulting PB/PS chimeric nucleic acid oligomer (dodecamer) was an antisense molecule having the base sequence d(G_{PB}C_{PS}A_{PB}T_{PS}T_{PS}G_{PB}G_{PB}T_{PS}A_{PB}T_{PS}T_{PB}C) (SEQ ID NO: 5).

### <Monomer Synthesis 2>

### (Synthesis of 2'-O-4'-C-Locked-H-boranophosphonate monomer 7a or 7c)

Hereinafter, a 2'-O-4'-C-Locked-H-boranophosphonate monomer is also referred to as Locked Nucleic Acid (hereinafter referred to as "LNA")-modified H-boranophosphonate monomer.

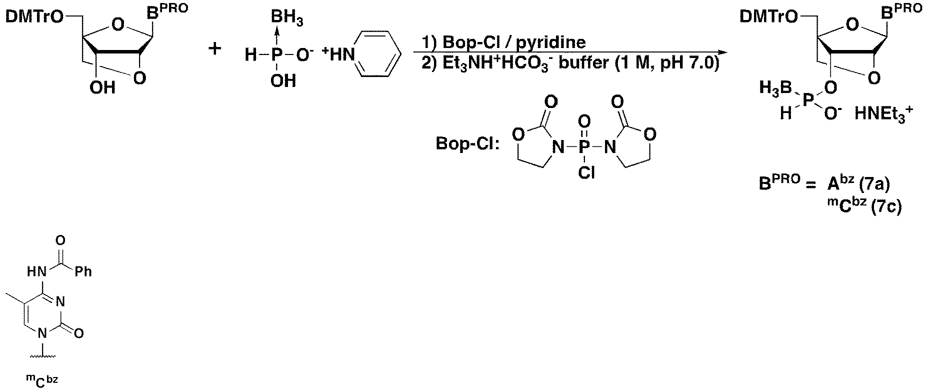

A nucleoside derivative shown on the far left in the scheme above (A: 0.69g, 1.0 mmol; or ^{m}C: 0.77g, 1.1 mmol; ^{m}C denotes 5-methylcytosine) and pyridinium H-boranophosphonate (A: 0.41 g, 2.0 mmol; or ^{m}C: 0.47 g, 2.3 mmol) were azeotropically dried under an argon atmosphere with pyridine (3 mL x 3) to be dissolved in pyridine (A: 10 mL; or ^{m}C: 11 mL). BopCl (A: 0.51 g, 2.0 mmol; or ^{m}C: 0.58 g, 2.3 mmol) was added to the resulting pyridine solution with stirring in an ice bath. Then, the reaction temperature was increased to room temperature and stirring was continued for 1 hour. The resulting solution was diluted with 60 mL of chloroform and then the resulting organic layer was washed with a 1.0 M TEAB buffer solution (pH 7.0) (80 mL x 3). The resulting aqueous layer was back-extracted with chloroform (40 mL x 3). The organic layer was collected and dehydrated over anhydrous sodium sulfate, and then, the solvent was distilled off from the organic layer under reduced pressure. The resulting residue was separated and purified by silica gel column chromatography (eluent: A: ethyl acetate-methanol-triethylamine (99:0.5:0.5, v/v/v) and then chloroform-methanol-triethylamine (98:2:0.5-96:4:0.5, v/v/v); ^{m}C: ethyl acetate-methanol-triethylamine (99.5:0.5:0.5, v/v/v) and then chloroform-methanol-triethylamine (99:1:0.5, v/v/v)), and the solvent was distilled off under reduced pressure to obtain LNA-modified H-boranophosphonate monomer 7a or 7c. 7a: yield 0.79 g, 0.92 mmol, yield 92%; 7c: yield 0.60 g, 0.67 mmol, yield 59%

### LNA-modified H-boranophosphonate monomer 7a

¹H-NMR(400MHz, CDCl₃) δ 9.50-9.10(br, 1H), 8.78 (s, 0.5H), 8.77(s, 0.5H), 8.40(s, 0.5H), 8.40(s, 0.5H), 8.06-7.96(m, 2H), 7.84-7.68(br, 0.5H), 7.64-7.42(m, 5H), 7.38-7.19(m, 7H), 6.89-6.83(m, 4H), 6.79-6.73(br, 0.5H), 6.17(s, 0.5H), 6.14(s, 0.5H), 4.95-4.89(m, 1H), 4.73(d, J=7.8Hz, 1H), 4.12-3.95(m, 2H), 3.83-3.73(m, 6H), 3.65-3.49(m, 2H), 2.91(q, J=7.5Hz, 6H), 1.19(t, J=7.5Hz, 9H), 1.0-0.0(br, 3H).

³¹P-NMR(162MHz, CDCl₃) δ 110-103(br).

### LNA-modified H-boranophosphonate monomer 7c

¹H-NMR (400MHz, CDCl₃) δ 8.35-8.28 (m, 2H), 7.90-7.86 (m, 1H), 7.55-7.42 (m, 5H), 7.41-7.30 (m, 6H), 7.27-7.22 (m, 1H), 6.89-6.81 (m, 4H), 6.84-6.78 (br, 0.5H)5.69 (s, 0.5H), 5.65 (s, 0.5H), 4.89 (d, J=6.4Hz, 0.5H), 4.70 (s, 0.5H), 4.67 (s, 0.5H), 4.58 (d, J=8.0Hz, 0.5H), 3.98-3.96 (m, 1H), 3.82-3.78 (m, 6H), 3.56-3.44 (m, 2H), 2.84 (q, J=7.2Hz, 6H), 1.87-1.82 (m, 3H), 1.18 (t, J=7.3Hz, 9H), 1.0-0.1 (3H).

³¹P-NMR (162MHz, CDCl₃) δ 111-104 (br).

### (Synthesis of 2'-O-4'-C-Locked-H-boranophosphonate monomer 7g)

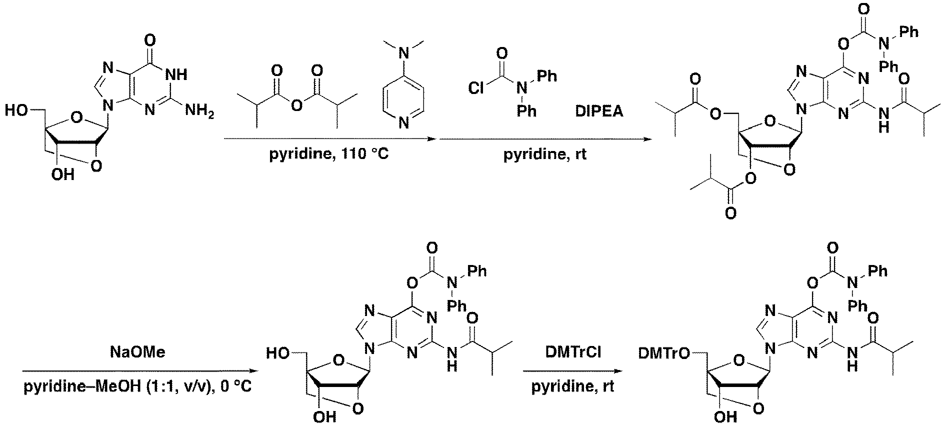

First, 2'-O-4'-C-Locked-guanosine (0.443 g, 1.5 mmol) was dissolved in 5 mL of pyridine under an argon atmosphere, 4-dimethylaminopyridine (0.189 g, 1.5 mmol) and isobutyric anhydride (1.25 mL, 7.5 mmol) were sequentially added thereto and reacted with each other at 110°C for 9 hours. Chloroform (20 mL) was added to the resulting mixture and washed with 1 M hydrochloric acid (20 mL), and the resulting aqueous layer was back-extracted with chloroform (20 mL). An organic layer was collected and washed with a saturated sodium bicarbonate aqueous solution (40 mL) twice, and an aqueous layer was back-extracted with chloroform (40 mL). The organic layer was collected, dried over sodium sulfate, and filtered, and the solvent was distilled off under reduced pressure. The resultant was used in the subsequent reaction without further purification. The resulting mixture was dissolved in pyridine (4 mL) under an argon atmosphere and diisopropylethylamine (0.4 mL, 2.25 mmol) and diphenylcarbamoyl chloride (0.69 g, 3.0 mmol) were sequentially added thereto and stirred at room temperature for 1 hour. Methanol (5 mL) was added thereto for quenching, chloroform (20 mL) was added thereto and washed with a saturated sodium bicarbonate aqueous solution (20mL) three times. The resulting aqueous layer was collected and back-extracted with chloroform (20 mL). The organic layer was collected, dried over sodium sulfate, and filtered, and the solvent was distilled off under reduced pressure. The resulting residue was purified by silica gel column chromatography (eluent: ethyl acetate-hexane (40:60 to 50:50, v/v)) to obtain 2'-O-4'-C-Locked-3',5'-O-diisobutyryl-2-N-isobutyryl-6-O-diphenylcarbamoyl-guanosine (0.859 g, 1.23 mmol, 82%).

A total amount of the resulting 2'-O-4'-C-Locked-3',5'-O-diisobutyryl-2-N-isobutyryl-6-O-diphenylcarbamoyl-guanosine was dissolved into a pyridine-methanol mixed solvent (1:1, v/v, 21 mL) and sodium methoxide (26.4 mg, 0.488 mmol) was added thereto stepwise in 4 portions at 0°C and stirred for 14 hours. The resultant was heated to room temperature, stirred for 4.5 hours, and then quenched by adding a cation exchange resin. The cation exchange resin was removed by filtering and the solvent was distilled off under reduced pressure. The resulting residue was purified by silica gel column chromatography (eluent: chloroform-methanol (100:0 to 100:4, v/v)) to obtain 2'-O-4'-C-Locked-2-N-isobutyryl-6-O-diphenylcarbamoyl-guanosine (0.401 g, 0.715 mmol, 58%).

A total amount of the resulting 2'-O-4'-C-Locked-2-N-isobutyryl-6-O-diphenylcarbamoyl-guanosine was dissolved into pyridine (7 mL) under an argon atmosphere, 4,4'-dimethoxytrityl chloride (0.266 g, 0.79 mmol) was added thereto and stirred at room temperature for 1.5 hours, and 4,4'-dimethoxytrityl chloride (61 mg, 0.18 mmol) was further added thereto and stirred for 40 min. Methanol (5 mL) was added thereto for quenching, chloroform (20 mL) was added thereto and washed with a saturated sodium bicarbonate aqueous solution (20 mL) three times. The resulting aqueous layer was collected and back-extracted with chloroform (20 mL). The organic layer was collected, dried over sodium sulfate, and filtered, and the solvent was distilled off under reduced pressure. The residue was purified by silica gel column chromatography (eluent: chloroform-methanol-pyridine (100:0:0.5 to 100:1.5:0.5, v/v/v)) to obtain 2'-O-4'-C-Locked-5'-O-(4,4'-dimethoxytrityl)-2-N-isobutyryl-6-O-diphenylcarbamoyl-guanosine (0.617 g, 0.451 mmol, 63%).

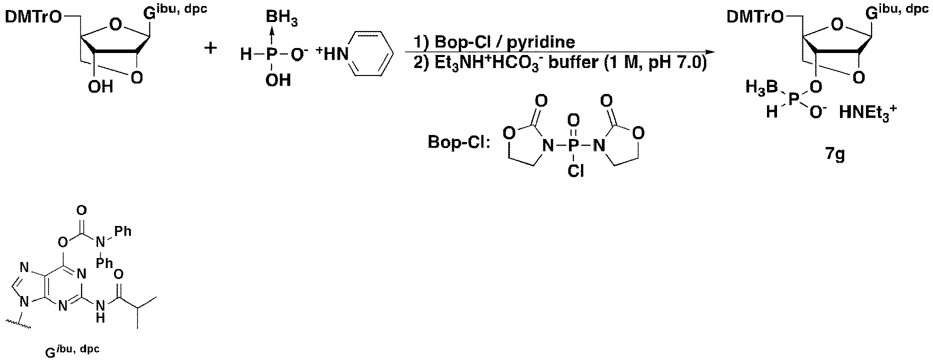

An LNA-modified guanosine derivative shown on the far left in the scheme above (0.39 g, 0.45 mmol) and pyridinium H-boranophosphonate (0.19 g, 0.90 mmol) were azeotropically dried under an argon atmosphere with pyridine (3 mL x 3) to be dissolved in pyridine (5 mL). BopCl (0.23 g, 0.90 mmol) was added to the resulting pyridine solution with stirring in an ice bath. Then, the reaction temperature was increased to room temperature and stirring was continued for 1 hour. The resulting solution was diluted with chloroform (30 mL) and then the organic layer was washed with a 1.0 M TEAB buffer solution (pH 7.0) (30 mL x 3). The resulting aqueous layer was back-extracted with chloroform (30 mL x 3). The organic layer was collected and dehydrated over anhydrous sodium sulfate, and then, the solvent was distilled off from the organic layer under reduced pressure. The resulting residue was separated and purified by silica gel column chromatography (eluent: ethyl acetate-triethylamine (100:0.5, v/v) and then chloroform-methanol-triethylamine (98:2:0.5, v/v/v)), and the solvent was distilled off under reduced pressure to obtain LNA-modified H-boranophosphonate monomer 7g (0.35 g, 0.92 mmol, 92%).

### LNA-modified H-boranophosphonate monomer 7g

¹H-NMR (400MHz, CDCl₃) δ 8.74 (s, 0.5H), 8.68 (s, 0.5H), 8.23 (s, 0.5H), 8.20 (s, 0.5H), 7.84-7.64 (m, 0.5H), 7.44-7.41 (m, 5H), 7.38-7.28 (m, 10H), 7.26-7.18 (m, 4H), 6.91-6.75 (m, 4H), 6.76-6.70 (br, 0.5H), 6.00 (s, 0.5H), 6.00 (s, 0.5H), 5.17 (d, J=6.4Hz, 0.5H), 4.96 (s, 0.5H), 4.89 (s, 0.5H), 4.82 (d, J=8.5Hz, 0.5H), 4.14-4.07 (m, 1.5H), 3.99-3.95 (m, 0.5H), 3.80-3.73 (m, 6H), 3.59-3.44 (m, 2H), 3.30-3.21 (m, 1H), 2.86 (q, J=7.3Hz, 6H), 1.27-1.19 (m, 6H), 1.12 (t, J=7.3Hz, 9H).

³¹P-NMR (162MHz, CDCl₃) δ 110-101 (br).

### (Synthesis of 2'-O-4'-C-Locked-H-boranophosphonate monomer 7t)

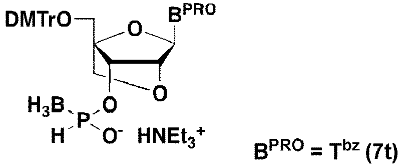

2'-O-4'-C-Locked-H-boranophosphonate monomer 7t was synthesized according to the synthesis method described in the publication (The Journal of Organic Chemistry, 2014, 79, 3465-3472) .

### (Synthesis of 2'-O-MOE-modified H-boranophosphonate monomer)

2'-O-MOE-modified H-boranophosphonate monomer was synthesized according to the below-described scheme.

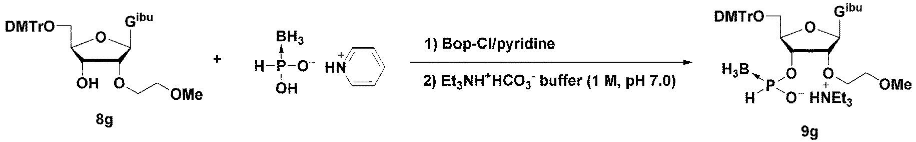

A guanosine derivative 8g derivative shown on the far left in the scheme above (0.722 g, 1.0 mmol) were azeotropically dried with pyridine (3 mL x 3) under an argon atmosphere and dissolved into 25 mL of pyridine along with pyridinium H-boranophosphonate (0.30 g, 2.0 mmol). BopCl (0.51 g, 2.0 mmol) was added to the resulting pyridine solution with stirring in an ice bath. Then, the reaction temperature was increased to room temperature and stirring was continued for 6 hours. The resulting solution was diluted with 30 mL of chloroform and then the organic layer was washed with a 1.0 M TEAB buffer solution (pH 7.0) (30 mL x 3). The resulting aqueous layer was back-extracted with chloroform (30 mL x 3). The organic layer was collected and dehydrated over anhydrous sodium sulfate, and then, the solvent was distilled off from the organic layer under reduced pressure. The resulting residue was separated and purified by silica gel column chromatography (eluent: ethyl acetate-methanol-triethylamine (100:0:1, v/v/v) and then chloroform-methanol-triethylamine (100:10:1, v/v/v)), and the solvent was distilled off under reduced pressure to obtain 2'-O-MOE-modified H-boranophosphonate monomer 9g. Yield: 0.57 g, 0.60 mmol, yield 60%

### 2'-O-MOE-modified H-boranophosphonate monomer 9g

¹H-NMR (400MHz, CDCl₃) δ 11.4-10.5 (br, 1H), 7.90 (s, 0.5H), 7.89 (s, 0.5H), 7.80-7.68 (br, 0.5H)7.42 (d, J=7.8Hz, 2H), 7.30 (d, J=8.2Hz, 4H), 7.24-7.14 (m, 3H), 6.93-6.87 (br, 0.5H), 6.78-6.74 (m, 4H), 5.97 (d, J=4.1Hz, 0.5H), 5.93 (d, J=4.6Hz, 0.5H), 5.40-5.27 (m, 1H), 4.82 (t, J=4.3Hz, 0.5H), 4.80 (t, J=4.3Hz, 0.5H), 4.43-4.34 (m, 1H)3.93-3.85 (m, 1H), 3.76-3.71 (m, 6H), 3.51-3.41 (m, 2H), 3.23 (d, J=7.3Hz, 3H), 2.91 (q, J=7.3Hz, 6H), 2.63-2.49 (m, 1H), 1.19 (t, J=7.3Hz, 9H), 1.14-1.03 (m, 6H), 0.9-0.2 (br, 3H).

³¹P-NMR (162MHz, CDCl₃) δ 108-103 (br).

### <Synthesis of nucleic acid oligomer by solid-phase method 2>

### (Solid-phase synthesis of N^{#}_{PB}T)

### [Synthesis Example 12]

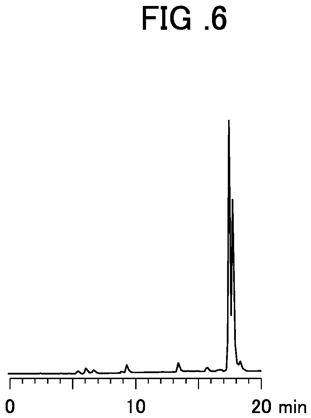

Hereinafter, N^{#} denotes a 2'-O-MOE-modified nucleotide unit. First, 5'-dimethoxytrityl-N³-benzoylthymidine (0.5 µmol) supported on a solid-phase support via a succinyl linker was detritylated with the addition of a 3% dichloroacetic acid/dichloromethane solution (4 x 15 s, each 1 mL) and then the solid-phase support was washed with acetonitrile and dichloromethane dried by molecular sieve. The solid-phase support was dried for 10 min and 2'-O-MOE-modified H-boranophosphonate monomer 9g (18.9 mg, 40 equivalents, 20 µmol)) and MNTP (22.3 mg, 100 equivalents, 50 µmol) were added to a reaction system. Then, 0.2 mL of an acetonitrile solution containing 2,6-lutidine serving as a base in the presence of argon was added as a reaction solvent and manually stirred slowly for 3 min. Thus, the monomer was condensed. After the 2'-O-MOE-modified H-boranophosphonate monomer was condensed, the solid-phase support was washed with acetonitrile (1 mL x 4) and dichloromethane (1 mL x 4) dried as described above and then a dimethoxytrityl group was deprotected with a 3% dichloroacetic acid/dichloromethane-triethylsilane (1:1, v/v) solution (4 x 15 s, each 1 mL). Then, the solid-phase support was washed with acetonitrile (1 mL x 4) and dichloromethane (1 mL x 4) dried as described above and dried for 10 min. The thus-formed H-boranophosphonate bond was oxidized for 90 min with the addition of a 0.1 M triethylamine/carbon tetrachloride-2,6-lutidine-water (5:12.5:1, v/v/v) solution to thereby be converted to a boranophosphate bond. The solid-phase support was washed with acetonitrile (1 mL x 4) and dichloromethane (1 mL x 4) dried as described above and reacted with a 25% ammonia water/ethanol (3:1, v/v, 5 mL) solution under the below-described conditions. Then, a base moiety of a nucleic acid was deprotected and a nucleic acid oligomer was cut from the solid-phase support (G^{#}_{PB}T: 50°C, 20h). After the reaction, the reaction solution was filtered and washed with ethanol. The solvent was distilled off under reduced pressure and the resulting crude product was analyzed by reverse-phase HPLC in the same manner as above. Results are shown in Table 5 and FIG. 6.

**[Table 5]**

| Entry | Monomer | Product | HPLC yield (%) |
|---|---|---|---|
| 1 | 9g | G^{#}_{PB}T | 97 |

In Table 5, an HPLC yield was calculated from an area ratio G^{#}_{PB}T / (T + G^{#}_{PB}T) based on the HPLC results.

### [Example 5] Synthesis of PB/PS-LNA-gapmer (dodecamer)

A nucleic acid oligomer having a base sequence complementary to that of the target nucleic acid (apoB protein) was synthesized in the same manner as in Example 2. As a solid-phase support, UnyLinker having a 5'-dimethoxytrityl group (Universal UnyLinker Support 1000 Å (ChemGenes), 0.5 µmol) was used. Furthermore, in addition to the monomer used in Example 2, an LNA-modified H-boranophosphonate compound having N⁴-benzoyl-5-methylcytosine, N⁶-benzoyladenine, N²-isobutyryl-O⁶-diphenylcarbamoylguanine, or N³-benzoylthymine as a base was used. Only a first base was introduced by a phosphoramidate method using an automated synthesizer (M-4 (NIHON TECHNO SERVICE CO., LTD.)) described in Step (A) below.

Step (A): A 3% trichloroacetic acid/dichloromethane solution was passed through a column for solid-phase synthesis filled with a solid-phase support four times and reacted for 40 seconds (each 10 seconds) to be detritylated. After completion of the reaction, the solid-phase support was washed with acetonitrile and dried by passing through an argon gas. Next, an acetonitrile solution into which a 5'-dimethoxytrityl-3'-cyanoethylphosphoroamidate unit (N⁴-Benzoyl-5-methyl-5'-O-(4,4'-dimethoxytrityl)-2'-O-4'-C-Locked-cytidin-3'-cyanoethyl Phosphoramidite) having N⁴-benzoyl-5-methylcytosine at a base moiety and LNA-modification at a sugar moiety was dissolved so as to have a concentration of 0.1 mol/L (hereinafter referred to as "amidate-dissolved solution") was passed through a column four times and reacted for 24 min (each 6 min) to thereby introduce an LNA-modified 5-methylcytidine derivative to the solid-phase support. At this time, 0.25 mol/L a 5-benzylthio-1-H-tetrazol-acetonitrile solution serving as an activating agent was also simultaneously passed through the column. A ratio of the amidate dissolved solution to the activating agent was 40:60 (v/v). After completion of the reaction, the solid-phase support was washed with acetonitrile and dried by passing through an argon gas. Then, the resultant was passed through an oxidizing agent (tetrahydrofuran-water-pyridine-iodine (66:12:22:0.6, v/v/v/w)) four times and reacted for 36 seconds (each 9 seconds) to thereby oxidize a phosphate moiety. After completion of the reaction, the solid-phase support was washed with acetonitrile and dried by passing through an argon gas. Then, the resultant was passed through a 1:1 mixed solution of a capping solution A (tetrahydrofuran-acetic anhydride-pyridine (8:1:1, v/v/v)) and a capping solution B (16% N-methylimidazole/tetrahydrofuran) four times and reacted for 80 seconds (each 20 seconds) to thereby cap a hydroxy group of an unreacted Unylinker. Finally, the solid-phase support was washed with acetonitrile and dried by passing through an argon gas. Thus, a solid-phase support of interest was obtained.

Step (B): Thereafter, a second base and later were produced by sequentially performing the deprotection reaction of a hydroxyl group having a protecting group and the condensation reaction repeatedly in the same manner as in Example 2 until a dodecamer was obtained. However, in this sequence, MNTP was used as a condensing agent for condensation using the LNA-modified H-boranophosphonate monomer and PyNTP was used as a condensing agent for condensation using a 2'-deoxyribonucleic acid type H-boranophosphonate monomer (condensing conditions for various monomers are shown in Table 6). Finally, an oxidizing reaction was performed to obtain a solid-phase support containing a PB/PS-LNA-gapmer (dodecamer).

**[Table 6]**

| Monomer | | Condensing agent | | Base | |
|---|---|---|---|---|---|
| 4a | 40 equivalents, 20 µmol | PyNTP | 100 equivalents, 50 µmol | 2,6-Lutidine | 0.6 M |
| 5g | 80 equivalents, 40 µmol | PyNTP | 200 equivalents, 100 µmol | - | |
| 5t | 40 equivalents, 20 µmol | PyNTP | 100 equivalents, 50 µmol | - | |
| 7a, 7c, 7g, 7t | 40 equivalents, 20 µmol | MNTP | 100 equivalents, 50 µmol | 2,6-Lutidine | 0.6 M |

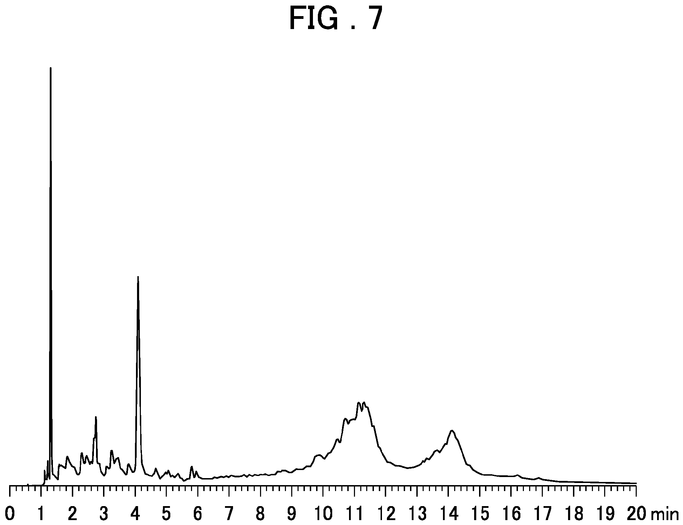

The resulting solid-phase support containing a PB/PS-LNA-gapmer (dodecamer) was reacted with 28% NH₃ water-ethanol (3:1, v/v) at 25°C (room temperature) for 6.5 hours and a nucleic acid oligomer was cut from the solid-phase support. The solid-phase support was filtered off through a 0.45 µm filter and a filtrate was further reacted at 55°C for 8 hours. Thus, a base moiety of a nucleic acid was deprotected. The solvent was distilled off from the resulting solution under reduced pressure and then reverse-phase UHPLC-MS analysis was performed. The reverse-phase UHPLC analysis was performed at 60°C using a 0.1 M hexafluoroisopropanol-0.008 M triethylamine buffer solution containing from 5 to 30% methanol as a mobile phase at a flow rate of 0.2 mL/min for 20 min. A chart of reverse-phase UHPLC for Example 5 is shown in FIG. 7. Purity: 18.7% (elution time: 11.3 min). LRMS (ESI-MS) Calcd for [M-4H]4-, 977.71; found, 977.89. [M-5H]5-, 781.97; found, 781.87

The resulting PB/PS-LNA-gapmer (dodecamer) was an antisense molecule having the base sequence G^{†}_{PB}mC^{†}_{PB}A^{†}_{PB}d(T_{PS}T_{PS}G_{PS}G_{PS}T_{PS}A_{PB})T^{†}_{PB}T^{†}_{PB}mC^{†} (SEQ ID NO: 6) .
(N^{t} denotes an LNA-modified nucleotide unit).

### [Example 6] Synthesis of PB/PS/PO-mixmer (dodecamer)

The monomer used in Example 2 and a 2'-O-MOE-modified H-boranophosphonate compound 9g having N²-isobutyrylguanine as a base were sequentially subjected to the deprotection reaction of a hydroxy group having a protecting group and the condensing reaction (condensing conditions for various monomers are shown in Table 7) repeatedly until a dodecamer was obtained so as to give a base sequence complementary to a base sequence of a target nucleic acid (apoB protein) and then to an oxidizing reaction in the same manner as in Example 2. Then, the resulting was treated with 25% NH₃ water-ethanol (3:1, v/v, 50°C, 20h) to deprotect a base moiety of a nucleic acid and a nucleic acid oligomer was cut from the solid-phase support, washed with ethanol (2 mL x 3), and the solvent was distilled off under reduced pressure to obtain a PB/PS/PO-mixmer (dodecamer).

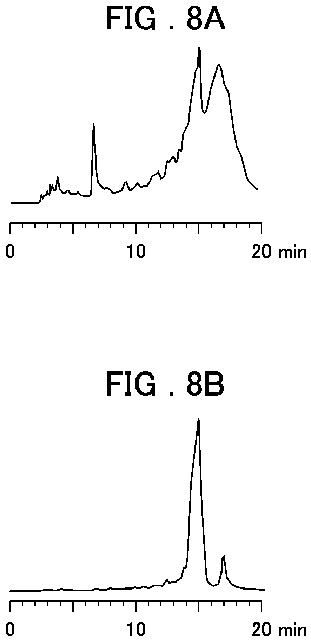

Then, separation and purification were performed by reverse-phase HPLC. The analysis by reverse-phase HPLC and the separation and purification were performed at 60°C using 0.4 M hexafluoroisopropanol-0.008 M triethylamine buffer solution containing from 10 to 45% methanol as a mobile phase at a flow rate of 0.5 mL/min for 20 min. Charts of reverse-phase HPLC before and after the separation and purification for Example 6 are shown in FIG. 8A and FIG. 8B, respectively. Yield: 20%. HRMS (ESI-TOF) Calcd for [M-4H]⁴⁻, 975.9717; found, 975.9670

The resulting PB/PS/PO-mixmer (dodecamer) was an antisense molecule having the base sequence G^{#}_{PB}d(C_{PO}A_{PS}T_{PB}T_{PO})G^{#}_{PB}G^{#}_{PB}d(T_{PB}A_{PS}T_{PS}T_{PB}C) (SEQ ID NO: 7) .

**[Table 7]**

| Monomer | | Condensing agent | | Base | |
|---|---|---|---|---|---|
| 3c, 3t | 40 equivalents, 20 µmol | MNTP | 100 equivalents, 50 µmol | 2,6-Lutidine | 0.6 M |
| 4t | 40 equivalents, 20 µmol | MNTP | 100 equivalents, 50 µmol | 2,6-Lutidine | 0.6 M |
| 5a | 40 equivalents, 20 µmol | PyNTP | 100 equivalents, 50 µmol | Quinoline | 1.8 M |
| 5t | 40 equivalents, 20 µmol | PyNTP | 100 equivalents, 50 µmol | - | |
| 9g | 40 equivalents, 20 µmol | MNTP | 100 equivalents, 50 µmol | 2,6-Lutidine | 0.6 M |

## Claims

1. A nucleic acid oligomer comprising:
a nucleotide unit represented by General Formula (1) below;
a nucleotide unit represented by General Formula (2) below; and
optionally a nucleotide unit represented by General Formula (3) below: 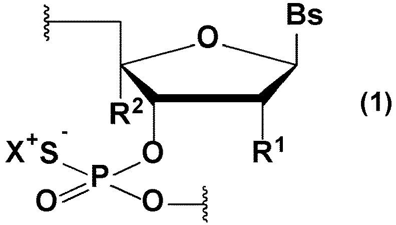 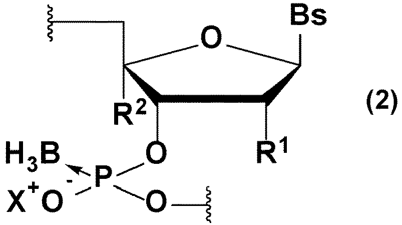 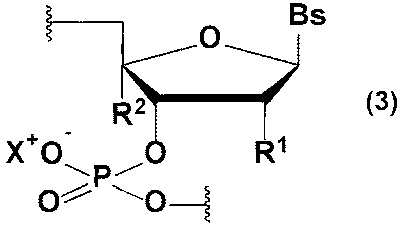
wherein R¹ denotes a hydrogen atom, a hydroxy group, an alkoxy group, an alkenyloxy group, an acyloxy group, a trialkylsilyloxy group, an alkoxyalkoxy group, a haloalkoxyalkoxy group, or a halogenyl group, R² denotes a hydrogen atom, or R¹ and R² bind to each other to form a five or more membered ring optionally having a substituent and optionally having a hetero atom, Bs denotes a nucleic acid base selected from the group consisting of adenine, guanine, cytosine, 5-methylcytosine, thymine, and uracil, the nucleic acid base optionally has a protecting group of the nucleic acid base, and X⁺ denotes a counter cation.

2. The nucleic acid oligomer according to claim 1, wherein R¹ denotes a hydrogen atom, a hydroxy group, an alkoxy group, an alkenyloxy group, an acyloxy group, a trialkylsilyloxy group, an alkoxyalkoxy group, a haloalkoxyalkoxy group, or a halogenyl group and R² denotes a hydrogen atom, or R¹ and R² bind to each other to form a divalent group represented by General Formula (4) below: 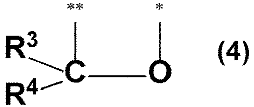 wherein R³ and R⁴ each independently denote a hydrogen atom or an alkyl group having 1 to 10 carbon atoms or bind to each other to form a ring, * and ** denote an atomic bond, an atomic bond represented by * is bonded to a carbon atom to which R¹ directly binds in General Formula (1), (2), or (3), and an atomic bond represented by ** is bonded to a carbon atom to which R² directly binds in General Formula (1), (2), or (3) .

3. A method for producing the nucleic acid oligomer according to claim 1 or 2, the method comprising:
sequentially condensing a nucleotide monomer selected from the group consisting of a compound represented by General Formula (5) below and a compound represented by General Formula (6) below or a nucleotide monomer selected from the group consisting of a compound represented by General Formula (5) below, a compound represented by General Formula (6) below, and a compound represented by General Formula (7) below to obtain a precursor nucleic acid oligomer comprising a nucleotide unit represented by General Formula (8) below and a nucleotide unit represented by General Formula (9) below and optionally comprising a nucleotide unit represented by General Formula (10) below; and
oxidizing the precursor nucleic acid oligomer with an oxidizing agent to obtain the nucleic acid oligomer according to claim 1 or 2: 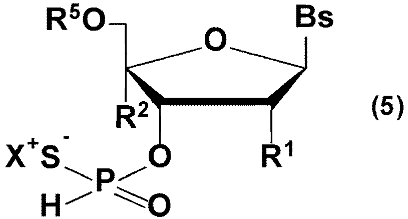 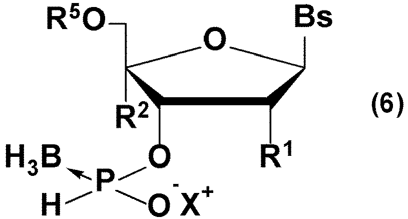 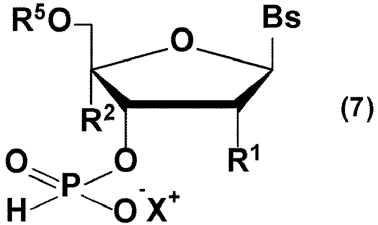
wherein R¹, R², Bs, and X⁺ are as described above and R⁵ denotes a hydrogen atom or a protecting group of a hydroxy group, 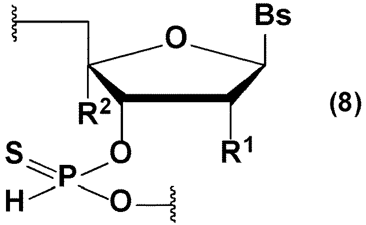 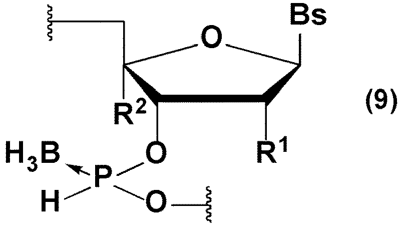 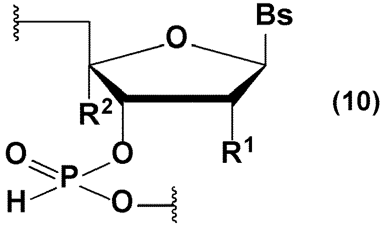
wherein R¹, R², and Bs are as described above.

4. The method according to claim 3, wherein at least the condensing is performed by a reaction using a solid-phase support.
